(19) Europäisches Patentamt
European Patent Office
Office européen des brevets

(11) EP 3 215 838 B1

(12) EUROPÄISCHE PATENTSCHRIFT

(45) Veröffentlichungstag und Bekanntmachung des Hinweises auf die Patenterteilung:
**03.10.2018 Patentblatt 2018/40**

(21) Anmeldenummer: **15797255.5**

(22) Anmeldetag: **04.11.2015**

(51) Int Cl.:
***G01N 27/414*** *(2006.01)* ***G01N 33/00*** *(2006.01)*

(86) Internationale Anmeldenummer:
**PCT/EP2015/002211**

(87) Internationale Veröffentlichungsnummer:
**WO 2016/070991 (12.05.2016 Gazette 2016/19)**

# (54) GASSENSOR UND GASMESSGERÄT ZUM NACHWEIS VOLATILER ORGANISCHER VERBINDUNGEN

GAS SENSOR AND GAS MEASURING DEVICE FOR DETECTING VOLATILE ORGANIC COMPOUNDS

CAPTEUR DE GAZ ET APPAREIL DE MESURE DE GAZ POUR DÉCELER DES COMPOSÉS ORGANIQUES VOLATILES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.11.2014 DE 102014016394**

(43) Veröffentlichungstag der Anmeldung:
**13.09.2017 Patentblatt 2017/37**

(73) Patentinhaber: **Dräger Safety AG & Co. KGaA
23560 Lübeck (DE)**

(72) Erfinder:
- **BÄTHER, Wolfgang
23558 Lübeck (DE)**
- **LEHMANN, Stefan
25436 Uetersen (DE)**

(74) Vertreter: **Kettenbeil, Roxane Henriette
Drägerwerk AG & Co. KGaA
Moislinger Allee 53/55
23558 Lübeck (DE)**

(56) Entgegenhaltungen:
**EP-A1- 2 006 668 DE-A1-102006 046 225
DE-A1-102012 022 136**

- **Hang Yuan ET AL: "Room temperature benzene gas detection using gated lateral BJT with assembled solvatochromic dye", , 23. Mai 2012 (2012-05-23), XP055244364, DOI: 10.5162/IMCS2012/1.5.5 Gefunden im Internet: URL:http://www.ama-science.org/proceedings /details/1160 [gefunden am 2016-01-25]**
- **ALTINDAL ET AL: "SOLUBLE DODECYLSULFANYLPHTHALOCYANINES AS SENSITIVE COATINGS FOR CHEMICAL SENSORS IN GAS PHASE", PROCEEDINGS OF THE 1998 IEEE INTERNATIONAL FREQUENCY CONTROL SYMPOSIUM. PASADENA, CA, MAY 27 - 29, 1998; [IEEE INTERNATIONAL FREQUENCY CONTROL SYMPOSIUM], NEW YORK, NY : IEEE, US, Bd. CONF. 52, 27. Mai 1998 (1998-05-27), Seiten 676-684, XP000847131, ISBN: 978-0-7803-4374-0**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 215 838 B1

## Beschreibung

Die vorliegende Erfindung betrifft einen Gassensor, ein Gasmessgerät und die Verwendung eines erfindungsgemäßen Gassensors zum Nachweis volatiler organischer Verbindungen.

Gassensoren werden typischerweise als elementarere Bestandteile von Gasmessgeräten verwendet. Beispielsweise werden sie in sogenannten personenbezogenen Gasmessgeräten (PAM = personal air monitor) eingesetzt. Mit solchen personenbezogenen Gasmessgeräten wird die Luft, die eine Person umgibt, kontinuierlich auf gefährliche Stoffe untersucht und gegebenenfalls eine entsprechende Warnung ausgegeben. An diese Gasmessgeräte werden hohe Anforderungen bezüglich der Quantifizierbarkeit der Messung, der Zuverlässigkeit, der Sicherheit, der Bedienbarkeit, der Empfindlichkeit und der Messzeit gestellt.

Gassensoren, die zum Nachweis von chemischen Verbindungen geeignet sind, bestehen grundsätzlich aus einer Kombination von Rezeptor und Transducer. Der Rezeptor - etwa eine Rezeptorelektrode - kann typischerweise auf molekularer Ebene mit den nachzuweisenden Analytmolekülen wechselwirken. Dabei ändert sich eine physikalisch-chemische Eigenschaft des Rezeptors, zum Beispiel die Austrittsarbeit der Rezeptoroberfläche. Der Transducer kann diese Änderung erfassen und in ein - beispielsweise elektrisches - Signal überführen. Das elektrische Signal kann dann wiederum zur Auslösung eines Alarmes oder dergleichen genutzt werden.

In diesem Zusammenhang sind zum Beispiel Gassensoren auf Halbleiterbasis bekannt, die aus einem Feldeffekttransistor (FET) und einem mit dem FET gekoppelten Kondensator bestehen (Capacitively-Controlled-Field Effect Transistor (CCFET)). Der Kondensator ist als Luftkapazität ausgebildet. Eine der beiden Kondensatorflächen wirkt als Rezeptor, wobei die Oberfläche der Kondensatorfläche (Rezeptoroberfläche) mit den nachzuweisenden Analytmolekülen wechselwirken kann. Das zu untersuchende Messgas durchströmt den Kondensator zwischen den Kondensatorflächen und bildet das Dielektrikum. Kommt es zu einer Wechselwirkung der Rezeptoroberfläche mit Analytmolekülen, die in dem Messgas vorhanden sind, so ändert sich die Kapazität des Kondensators. Diese Kapazitätsänderung kann mit Hilfe des FET erfasst und in ein elektrisches Signal umgewandelt werden.

US 4,411,741 A sieht in diesem Zusammenhang einen Gassensor vor, bei welchem eine Rezeptorelektrode gegenüber der Gate-Elektrode eines FET angeordnet ist und von der Gate-Elektrode durch einen Luftspalt getrennt ist. Die Gate-Elektrode des FET und die Rezeptorelektrode bilden dabei die Kondensatorflächen.

Auch DE 43 33 875 A1 sieht einen Halbleiter-Gassensor mit einem FET vor. Der auswertende FET und der Luftspalt, in dem die gasempfindlichen Schicht ausgebildet ist, d.h. der Kondensator, sind hier räumlich von einander getrennt angeordnet, aber dennoch elektrisch miteinander gekoppelt. Der FET weist eine Gate-Elektrode auf, die leitend mit einer Sensorelektrode verbunden ist. Gegenüber der Sensorelektrode ist eine gassensitive Schicht angeordnet. Die gassensitive Schicht ist durch einen Luftspalt von der Sensorelektrode beabstandet und über den Luftspalt kapazitiv an die Sensorelektrode gekoppelt.

EP 2 006 668 A1 sieht ebenfalls einen solchen Sensor vor. Dabei ist die gassensitive Schicht von einer zusätzlichen Schutzschicht bedeckt. Diese zusätzliche Schutzschicht ist haftend mit der gassensitiven Schicht verbunden aber durchlässig für das Zielgas. Sie dient dazu, stark nicht-lineare Änderungen des Messsignales, die bei bestimmten Kombinationen von gassensitiver Schicht und Analyt trotz mehr oder weniger linearer Änderung der Zielgaskonzentration auftreten können, derart zu modifizieren, dass die Änderung des Messsignales in etwa der Änderung der Zielgaskonzentration entspricht.

Typische Materialien, aus denen die Rezeptorschicht solcher bekannter Gassensoren bestehen kann, sind Metalle, Halbleiter, Halbleiterverbindungen oder Metallverbindungen, wie etwa Platin, Palladium, Titannitrid, Kupfer-Phthalocyanin, Bariumtitanat, Zinndioxid, Silberoxid, Cobaltoxid, Chrom-Titan-Oxid, Gold, Kaliumjodid oder auch Germanium. Von dem jeweils verwendeten Material hängt es ab, welche Analyte nachweisbar sind. So ist beispielsweise Wasserstoff mit Hilfe von Platin oder Palladium nachweisbar, während $NO_2$ und andere anorganische Gase unter anderem mit Rezeptorschichten aus Cobaltoxid, Zinndioxid oder Kupfer-Phthalocyanin nachweisbar sind. Problematisch ist dabei häufig der Nachweis von sogenannten volatilen organischen Verbindungen, insbesondere wenn sie sich im Allgemeinen relativ inert verhalten wie z.B. Benzol. Ein Grund hierfür ist die häufig gar nicht oder nur eingeschränkt stattfindende Wechselwirkung, die solche organischen Verbindungen mit den als Rezeptorschicht verwendeten Materialien zeigen. Als Alternative werden daher häufig optische Sensoren zum Nachweis solcher Verbindungen verwendet. Diese können jedoch sehr empfindlich gegenüber Stößen und anderen mechanischen Einwirkungen sein. Zudem sind sie häufig relativ groß. Nichtsdestotrotz, in Room temperature benzene gas detection using gated lateral BJT with assembled solvatochromic dye präsentierten auf der IMCS 2012 Konferenz Yuan H. et al einen Benzol Gassensor mit einem Farbstoff, der auf dem floating gate eines Transistors mittels SAMs immobilisiert war.

Ausgehend davon ist es unter anderem Ziel der Erfindung, diese und weitere Nachteile des Standes der Technik zu überwinden und einen verbesserten Gassensor zu schaffen. Beispielsweise soll ein Gassensor bereitgestellt werden, der zum Einsatz in einem PAM geeignet sein kann. Wünschenswert ist dabei, dass mit Hilfe eines solchen Sensors volatile organische Verbindungen, insbesondere Benzol, sicher und zuverlässig nachweisbar sein können.

Zur Lösung dieser Aufgabe schlägt die Erfindung einen Gassensor entsprechend Anspruch 1, sowie ein Gasmessgerät mit einem solchen Gassensor und die Verwendung eines solchen Gassensors zum Nachweis von volatilen organischen Verbindungen gemäß den Ansprüchen 20 und 21 vor. Ausgestaltungen sind jeweils Gegenstand der abhängigen Ansprüche.

Dabei weist der Gassensor einen Messkanal mit einem Gaseinlass und einem Gasauslass, mindestens eine Rezeptorschicht, eine Referenzelektrode und eine Auswerteeinrichtung aufweist,

- wobei die Referenzelektrode kapazitiv mit der Rezeptorschicht gekoppelt ist,
- wobei die Referenzelektrode elektrisch leitend mit der Auswerteeinrichtung verbunden ist,
- wobei die Rezeptorschicht in dem Messkanal ausgebildet ist,
- wobei der Messkanal eine dielektrische Schicht zwischen der Rezeptorschicht und der Referenzelektrode bildet, und
- wobei die Rezeptorschicht einen Träger und eine Analytbindungsschicht aufweist

**sieht die Erfindung vor,**
dass die Analytbindungsschicht eine selbstorganisierende monomolekulare Schicht ist, die aus Molekülen gemäß der allgemeinen Formel

$$R^1\text{-}R^2\text{-}X$$

besteht, wobei $R^1$ eine Kopplungsgruppe ist, ausgewählt aus der Gruppe enthaltend Sulfid, Disulfid, Sulfinyl, Sulfino, Sulfo, Carbonothiol, Thiosulfat, Thiocyanat, Isothiocyanat, bevorzugt Sulfid oder Thiosulfat, und wobei die Moleküle der selbstorganisierenden monomolekularen Schicht jeweils über $R^1$ an den Träger gekoppelt sind;
wobei der Träger eine Schicht aus Metall ist, wobei das Metall ausgewählt ist aus der Gruppe enthaltend Gold, Platin, Palladium, Silber und Kupfer;
wobei $R^2$ ein Spacer ist, ausgewählt aus der Gruppe enthaltend Alkan, Alken, Alkin, Heteroalkan, Heteroalken, Heteroalkin, substituierte Alkane, substituierte Alkene, substituierte Alkine, substituierte Heteroalkane, substituierte Heteroalkene, substituierte Heteroalkine, Ether, Amine; und
wobei X eine organische oder metall-organische Gruppe ist, die mit einem Analytmolekül in Wechselwirkung treten kann, insbesondere eine organische oder metall-organische Gruppe mit wenigstens einem delokalisierten $\pi$-System, und wobei die Spacer $R^2$ benachbarter Moleküle kovalent miteinander verbunden sind.

Mit anderen Worten, die Analytbindungsschicht kann eine selbstorganisierende monomolekulare Schicht sein, die aus Molekülen gemäß der allgemeinen Formel

$$R^1\text{-}R^2\text{-}X$$

besteht, wobei $R^1$ eine Kopplungsgruppe ist, ausgewählt aus der Gruppe enthaltend Sulfid, Disulfid, Sulfinyl, Sulfino, Sulfo, Carbonothiol, Thiosulfat, Thiocyanat, Isothiocyanat, bevorzugt Sulfid oder Thiosulfat, und wobei die Moleküle der selbstorganisierenden monomolekularen Schicht jeweils über $R^1$ an den Träger gekoppelt sind; wobei der Träger eine Schicht aus Metall ist, wobei das Metall ausgewählt ist aus der Gruppe enthaltend Gold, Platin, Palladium, Silber und Kupfer; wobei $R^2$ ein Spacer ist, ausgewählt aus der Gruppe enthaltend Alkan, Alken, Alkin, Heteroalkan, Heteroalken, Heteroalkin, substituierte Alkane, substituierte Alkene, substituierte Alkine, substituierte Heteroalkane, substituierte Heteroalkene, substituierte Heteroalkine, Ether, Amine; und wobei X eine organische oder metall-organische Gruppe mit wenigstens einem delokalisierten $\pi$-System ist, und wobei die Spacer $R^2$ benachbarter Moleküle kovalent miteinander verbunden sind.

Die Analytbindungsschicht kann auch eine selbstorganisierende monomolekulare Schicht sein, die aus Molekülen gemäß der allgemeinen Formel

$$R^1\text{-}R^2\text{-}X$$

besteht, wobei $R^1$ eine Kopplungsgruppe ist, ausgewählt aus der Gruppe enthaltend Sulfid oder Thiosulfat, und wobei die Moleküle der selbstorganisierenden monomolekularen Schicht jeweils über $R^1$ an den Träger gekoppelt sind; wobei der Träger eine Schicht aus Metall ist, wobei das Metall ausgewählt ist aus der Gruppe enthaltend Gold, Platin, Palladium, Silber und Kupfer; wobei $R^2$ ein Spacer ist, ausgewählt aus der Gruppe enthaltend Alkan, Alken, Alkin, Heteroalkan, Heteroalken, Heteroalkin, substituierte Alkane, substituierte Alkene, substituierte Alkine, substituierte Heteroalkane, substituierte Heteroalkene, substituierte Heteroalkine, Ether, Amine; und wobei X eine organische oder metall-organische

Gruppe mit wenigstens einem delokalisierten π-System ist, und wobei die Spacer $R^2$ benachbarter Moleküle kovalent miteinander verbunden sind.

Ein zu untersuchendes Gas (Messgas) kann mithin in jedem Fall durch den Gaseinlass in den Messkanal des Gassensors einströmen und durch den Gasauslass wieder aus dem Messkanal herausströmen. Mithin durchströmt das Messgas den Messkanal als Messgasstrom. Die Erfindung umfasst dabei sowohl einen Gassensor, der gerade von einem Messgasstrom durchströmt wird, als auch einen Gassensor, ohne Messgasstrom. Letzteres kann beispielsweise der Fall sein, wenn der Gaseinlass und/oder der Gasauslass vor der Montage des Gassensors verschlossen sind, etwa zu Transportzwecken oder dergleichen. Der Messkanal weist in jedem Fall eine Innenwand auf, die das Innere des Messkanales begrenzt. Erfindungsgemäß ist die Rezeptorschicht ein Teil dieser Innenwand. Insofern ist die Rezeptorschicht ein Teil des Messkanals. Das durch den Messkanal strömende Messgas strömt an der Rezeptorschicht entlang. Die Rezeptorschicht bildet dabei eine Oberfläche, die mit einem nachzuweisenden Analyten wechselwirken kann. Der Analyt ist typischerweise in dem Messgas enthalten, welches als Messgasstrom durch den Messkanal strömt. Auch die Referenzelektrode kann ein Teil der Innenwand sein. Der Messgasstrom bzw. das Messgas kann mithin zwischen der Rezeptorschicht und der Referenzelektrode durch den Messkanal strömen.

Die Rezeptorschicht stellt in diesem Zusammenhang eine Fläche eines Kondensators dar. Der Kondensator wird dabei bevorzugt durch die Rezeptorschicht, die Referenzelektrode und den als Dielektrikum wirkenden Messgasstrom bzw. das als Dielektrikum wirkende Volumen des Messkanales zwischen der Rezeptorschicht und der Referenzelektrode gebildet. Die Referenzelektrode ist insofern mit der Rezeptorschicht kapazitiv gekoppelt. Beispielsweise können die Rezeptorschicht und die Referenzelektrode an einander gegenüberliegenden Seiten des Messkanals ausgebildet sein, bevorzugt an gegenüberliegenden Seiten der Innenseite des Messkanals. Der Messkanal wird von dem Messgas, d.h. von dem zu untersuchenden Gas, durchströmt, so dass der Messgasstrom zwischen der Rezeptorschicht und der Referenzelektrode hindurchströmt. Der Messkanal bildet mithin ein Volumen zwischen den beiden Flächen des Kondensators, das sowohl wenn der Messkanal von Messgas durchströmt wird, als auch wenn der Messkanal leer ist - d.h. in dem eher unwahrscheinlichen Fall, dass z.B. ein Vakuum in dem Messkanal herrscht - als Dielektrikum wirken kann. Insofern wirkt der Messkanal als Dielektrikum zwischen den Kondensatorflächen. D.h. der Messkanal bildet eine dielektrische Schicht. Günstig ist es dabei, wenn der Messkanal beheizbar ist. Außerdem ist es vorteilhaft, wenn der Messkanal druckdicht ist.

Der Messkanal kann von einem Deckel und einer Isolatorschicht begrenzt sein. Ein solcher Deckel und eine solche Isolatorschicht begrenzen wenigstens einen Teil des Volumens des Messkanals. Die Rezeptorschicht kann auf dem Deckel ausgebildet sein. Die Referenzelektrode kann in der Isolatorschicht eingebettet sein. Vorstellbar ist in diesem Zusammenhang, dass die Auswerteeinrichtung ebenfalls in der Isolatorschicht eingebettet ist.

Die Auswerteeinheit ist entweder strom- oder spannungsgesteuert, bevorzugt jedoch spannungsgesteuert. Eine spannungsgesteuerte Auswerteeinrichtung kann in diesem Zusammenhang beispielsweise ein Transistor oder auch ein anderes spannungsgesteuertes elektronisches Bauteil, etwa ein spannungsgesteuerter Oszillator, oder dergleichen sein. Wesentlich ist dabei, dass die spannungsgesteuerte Auswerteeinheit Kapazitätsänderungen des Kondensators erkennen und verarbeiten kann, ohne dass ein Stromfluss notwendig ist. Kommt es zu einer Wechselwirkung eines Analyten mit der Rezeptorschicht, so ändert sich die Austrittsarbeit (d.h. diejenige Energie, die aufgewandt werden muss, um ein Elektron aus einer entsprechenden Materialschicht zu lösen) an der Rezeptorschicht und in der Folge kommt es zu einer Kapazitätsänderung des Kondensators. Letztere kann insbesondere dann von der spannungsgesteuerten Auswerteeinheit erkannt werden, wenn die Referenzelektrode elektrisch leitend mit ihr verbunden ist.

Es hat sich gezeigt, dass es besonders günstig ist, wenn die Rezeptorschicht aus einem Träger und einer Analytbindungsschicht besteht. Dabei kann die Analytbindungsschicht sowohl mit dem nachzuweisenden Analyten als auch mit dem Träger wechselwirken. Eine Wechselwirkung der Analytbindungsschicht mit einem Analyten führt dabei bevorzugt zu einer Änderung der Austrittsarbeit des Trägers und in der Folge zu der bereits oben beschriebenen nachweisbaren Kapazitätsänderung. Besonders vorteilhaft ist es in diesem Zusammenhang, wenn die Analytbindungsschicht eine selbstorganisierende monomolekulare Schicht (self assembling monolayer, SAM) ist. Diese SAM bildet diejenige Schicht, die mit dem nachzuweisenden Analyten wechselwirkt und an die der Analyt bevorzugt reversibel binden kann (sich anlagern kann).

Eine selbstorganisierende monomolekulare Schicht (SAM) ist typischerweise eine Schicht, deren Dicke einem Molekül des schichtbildenden Materials entspricht. Das schichtbildende Material ist dabei üblicherweise eine organische Verbindung. Die Moleküle einer SAM ordnen sich spontan durch Adsorption an einer Oberfläche an und richten sich durch Wechselwirkung miteinander in einer mehr oder weniger geordneten Art und Weise zueinander aus. Erfindungsgemäß bildet der Träger der Rezeptorschicht die Oberfläche, auf der sich die SAM anordnet und ausrichtet. Der Träger und die Analytbindungsschicht, d.h. die SAM, sind dabei bevorzugt kovalent miteinander verbunden.

Der erfindungsgemäße Gassensor zeichnet sich weiterhin besonders vorteilhaft dadurch aus, dass die Rezeptorschicht aus einem Träger auf dessen Oberfläche eine SAM ausgebildet ist, deren Moleküle der Formel $R^1$-$R^2$-X entsprechend, besteht. Während die Kopplungsgruppe $R^1$ dazu dient, die Moleküle der SAM an den Träger zu binden und sie auf dem Träger anzuordnen, dient der Spacer $R^2$ dazu, die Moleküle untereinander in einer gewissen Ordnung

zu halten. Dies wird dadurch erzielt, dass die Spacer $R^2$ benachbarter Moleküle kovalent miteinander verbunden sind. X stellt in diesem Zusammenhang die reaktive Gruppe des Moleküls $R^1$-$R^2$-X dar. Mit dieser reaktiven Gruppe können nachzuweisende Analyte wechselwirken. Die reaktive Gruppe kann zudem - wie auch der Spacer - Einfluss auf die Ordnung der Moleküle des SAM haben.

Ein besonderer erfindungsgemäßer Vorteil liegt darin, dass X eine organische oder metall-organische Gruppe mit wenigstens einem delokalisierten $\pi$-System sein kann. Auf diese Weise können insbesondere auch relativ inerte volatile organische Verbindungen, wie etwa Benzol, mit der SAM wechselwirken.

Unter einem delokalisierten $\pi$-System werden dabei typischerweise Molekülorbitale verstanden, die sich über mehrere C-Atome erstrecken und in denen sich die Elektronen relativ frei bewegen können. Charakteristisch für delokalisierte $\pi$-Systeme sind mesomere Effekte. Ein mesomerer Effekt ist dabei die Beeinflussung der Elektronenverteilung in einem solchen System durch Atome, die $\pi$-Bindungselektronen an sich ziehen (elektronenziehender Effekt, -M-Effekt) oder $\pi$-Bindungselektronen zur Verfügung stellen (elektronenschiebender Effekt, +M-Effekt).

Dieses $\pi$-System kann dabei zum Beispiel mit einem delokalisierten $\pi$-System eines entsprechenden Analyten wechselwirken. Dabei kann sich der Analyt an die reaktive Gruppe X anlagern, und eine Verschiebung der $\pi$-Elektronen bewirken. Die Verschiebung der $\pi$-Elektronen führt zu einer Änderung der Dipolmomente der beteiligten Moleküle und in der Folge zu einer gewünschten und messbaren Änderung der Austrittsarbeit.

Die Anlagerung des Analyten an die Gruppe X erfolgt bevorzugt über intermolekulare Wechselwirkungen, zum Beispiel durch das Wirken von Van-der-Waals-Kräften oder durch Wasserstoffbrücken-Bindungen. Denkbar ist auch, dass die intermolekulare Wechselwirkung zwischen den Analytmolekülen und der reaktiven Gruppe X zu einer Stabilisierung eines Komplexes führt. Die Verschiebung der $\pi$-Elektronen insbesondere in der Gruppe X bzw. die Änderung der Dipolmomente der beteiligten Moleküle kann mittels elektronenschiebender oder elektronenziehender Effekte durch Substituenten an der Gruppe X über den Spacer $R^2$ und die Kopplungsgruppe $R^1$ an den Träger weitergegeben werden, wodurch es letztlich zur Änderung der Austrittsarbeit kommt.

Günstig ist es dabei, wenn $R^2$ ein Spacer ist, der ausgewählt ist aus der Gruppe enthaltend Alkan, Alken, Alkin, Heteroalkan, Heteroalken, Heteroalkin, substituierte Alkane, substituierte Alkene, substituierte Alkine, substituierte Heteroalkane, substituierte Heteroalkene, substituierte Heteroalkine, Ether oder Amine. Mit Hilfe eines solchen Spacers ist der Abstand zwischen dem Träger bzw. der Kopplungsgruppe und der reaktiven Gruppe bestimmbar. Es ist insofern vorteilhaft, wenn der Spacer ein lineares Molekül bzw. eine lineare Molekülgruppe ist. Beispielsweise kann der Spacer eine lineare Atomkette als Rückgrat (Backbone) aufweisen, die aus Kohlenstoffatomen oder aus einer Mischung aus Kohlenstoff-, Sauerstoff- und Stickstoffatomen besteht. Die Atome der linearen Atomkette können dabei über Einfach-, Doppel- und/oder Dreifachbindungen miteinander verbunden sein. Insofern kann der Spacer beispielsweise ein Alkan, Alken, Alkin oder auch ein Heteroalkan, Heteroalken oder Heteroalkin sein. An das Rückgrat können zudem Substituenten gebunden sein, die beispielsweise die Übertragung der Dipoländerung von der reaktiven Gruppe X an die Kopplungsgruppe $R^1$ unterstützen. Denkbar ist auch, dass diese Substituenten zu Wechselwirkungen zwischen benachbarten Spacern führen. Auf diese Weise ist auch die Ordnung und Stabilität der SAM mit Hilfe des Spacers unterstützbar bzw. beeinflussbar. Mithin kann es auch vorteilhaft sein, wenn der Spacer ein substituiertes Alkan, substituiertes Alken, substituiertes Alkin oder auch ein substituiertes Heteroalkan, substituiertes Heteroalken oder substituiertes Heteroalkin ist.

Es hat sich gezeigt, dass es zudem vorteilhaft ist, wenn $R^1$ eine Kopplungsgruppe ist, die ausgewählt ist aus der Gruppe enthaltend Sulfid, Disulfid, Sulfinyl, Sulfino, Sulfo, Carbonothiol, Thiocyanat, Isothiocyanat, bevorzugt Sulfid oder Thiosulfat. Mit Hilfe einer solchen Kopplungsgruppe können die einzelnen Moleküle der SAM mit den an der Oberfläche des Trägers angeordneten Metallatomen wechselwirken und eine kovalente Bindung eingehen. Es besonders günstig ist, wenn die Kopplungsgruppe wenigstens ein Schwefelatom aufweist, welches die Bindung der Kopplungsgruppe $R^1$ an die Oberfläche des Trägers bewirkt. Dabei geht bevorzugt jede Kopplungsgruppe $R^1$ eine Bindung mit genau einem Oberflächenatom des Trägers ein. Man erkennt insofern, dass es günstig ist, wenn der Träger eine Schicht aus Metall ist, wobei das Metall ausgewählt ist aus der Gruppe enthaltend Gold, Platin, Palladium, Silber und Kupfer.

In jedem Fall ist es bei einem erfindungsgemäßen Gassensor günstig, wenn der Träger eine Schicht aus Gold ist. Auf einer Goldschicht können sich besonders gut SAMs anordnen. Mithin ist ein solcher Träger besonders gut geeignet, um mit einer SAM als Analytbindungsschicht beschichtet zu werden. Gold kann zudem besonders gut mit Schwefelverbindungen wechselwirken. Eine Goldschicht kann außerdem in hoher Reinheit hergestellt werden und ist insbesondere gegenüber Oxidation unempfindlich.

Vorteilhaft ist es zudem, wenn die Kopplungsgruppe $R^1$ kovalent mit dem Spacer $R^2$ und mit dem Träger verbunden ist. Die Kopplungsgruppe $R^1$ kann auf diese Weise die Anordnung der einzelnen Moleküle auf dem Träger steuern und die SAM-Moleküle in ihrer Position auf dem Träger verankern.

Für eine solche Bindung der SAM-Moleküle ist es günstig, wenn die Kopplungsgruppe $R^1$ wenigstens eine Schwefelbrücke zwischen dem Spacer und dem Träger ausbildet. Insbesondere wenn der Träger aus Gold besteht, ist eine Kopplung der SAM-Moleküle über eine Schwefelbrücke vorteilhaft. Es ist insofern günstig, wenn $R^1$ ausgewählt ist aus der Gruppe enthaltend Sulfid oder Thiosulfat. Insbesondere Thiolgruppen haben eine hohe Affinität zu Edelmetallen,

insbesondere zu Gold. Insofern ist es ganz besonders vorteilhaft, wenn $R^1$ ein Sulfid-Rest ist.

In einem weiteren Aspekt ist es vorteilhaft, dass $R^2$ ausgewählt ist aus der Gruppe enthaltend Alkane, Alkene, Alkine, substituierte Alkane, substituierte Alkene, substituierte Alkine, Ether, Amine wobei die Substituenten der substituierten Alkane, Alkene oder Alkine ausgewählt sind aus der Gruppe enthaltend Wasserstoff, Alkyl oder Aryl. Alternativ ist auch denkbar, dass $R^2$ ausgewählt ist aus der Gruppe enthaltend Heteroalkene, Heteroalkine, substituierte Heteroalkene und substituierte Heteroalkine.

In jedem Fall ist es günstig, wenn die Länge der Atomkette, die das Rückgrat des Spacers $R^2$ bildet, kleiner oder gleich 40 Atome ist. Bevorzugt ist es dabei, wenn die Länge der Atomkette 5, 6, 7, 8, 9, 10, 11, 12, 13, 14 oder 15 Atome beträgt, besonders bevorzugt 6, 7, 8, 9, 10, 11 oder 12 Atome, ganz besonders bevorzugt 6, 7, 8, 9 oder 10 Atome.

Man erkennt insofern, dass es günstig ist, $R^2$ eine lineare Molekülgruppe entsprechend der Formel $(Y)_n$ mit $n \in \{0, ..., 40\}_z$ ist, wobei jedes Y unabhängig von den übrigen Y des jeweiligen $R^2$ ausgewählt ist aus der Gruppe enthaltend $CH_2$, CH, C, $CR^3$, O, N, $NR^3$, und wobei $R^3$ ausgewählt ist aus der Gruppe enthaltend H, Alkan, Alken, Alkin oder Aromat. Y bezeichnet dabei die Elemente bzw. Reste von $R^2$, die die lineare Atomkette des Spacer-Rückgrates bilden. So ist beispielsweise vorstellbar, dass $R^2$ eine Molekülgruppe entsprechend der Formel $(CH_2)_4(CH)_2(CH_2)_6$ ist, was entsprechend der oben genannten Formel als $(Y)_{12}$ dargestellt würde, wobei Y in diesem Fall ausgewählt wäre aus $CH_2$ und CH. Ein Molekül einer entsprechenden SAM könnte in diesem Fall gemäß der Formel als $R^1$-$R^2$-X entsprechend als $R^1$-$(Y)_{12}$-X wobei Y $CH_2$ oder CH ist bzw. als $R^1$-$(CH_2)_4(CH)_2(CH_2)_6$-X dargestellt werden. Es versteht sich von selbst, dass dieses Beispiel nur zur Erläuterung der Formelschreibweise dient und keineswegs eine Einschränkung der vorliegenden Erfindung darstellt. Die Menge der möglichen und denkbaren Kombinationen für die Reste Y des Spacers $R^2$ ergibt sich vielmehr aus der Menge der verschiedenen denkbaren Längen der von Y gebildeten Ketten und der Auswahl der für Y gemäß dem oben ausgeführten denkbaren Reste. Besonders bevorzugt ist es dabei, wenn $n \in \{5, ..., 15\}_z$, bevorzugt $n \in \{6, ..., 10\}_z$ ist. Die Formel

$$R^1\text{-}R^2\text{-}X$$

der Moleküle der SAM kann mithin bei einer bevorzugten Ausführungsvariante auch als

$$R^1\text{-}(Y)_n\text{-}X$$

mit $n \in \{0, ..., 20\}_z$, bevorzugt $n \in \{5, ..., 15\}_z$, besonders bevorzugt $n \in \{6, ..., 10\}_z$ dargestellt werden.

Vorteilhaft ist es, wenn $R^2$ derart ausgewählt ist, dass die Spacer benachbarter Moleküle durch van-der Waals-Kräfte miteinander wechselwirken. Auf diese Weise können die Spacer die SAM besonders gut stabilisieren. Insbesondere können die Spacer auf diese Weise dazu beitragen, dass die reaktive Gruppen X benachbarter Moleküle in einer günstigen Position zu einander ausgerichtet sind, so dass nachzuweisende Analyte mit der SAM in eine effektive Wechselwirkung treten können. Beispielsweise können die reaktiven Gruppen X derart ausgerichtet sein, dass ein entsprechender nachzuweisender Analyt zwischen die reaktiven Gruppen X zweier benachbarter Moleküle interkaliert, wobei diese Interkalation eine Änderung des Dipolmomentes der Moleküle der SAM bewirkt. Um dies zu erreichen, sind die Spacer benachbarter Moleküle kovalent miteinander verbunden. Eine kovalente Verbindung kann dabei sowohl entlang der gesamten Kette von $R^2$ als auch zwischen einzelnen Abschnitten der Kette von $R^2$ ausgebildet sein.

Man erkennt insgesamt, dass die SAM verschiedenste Kombinationen der Gruppen $R^1$ und $R^2$ aufweisen können. In einer besonders bevorzugten Ausführungsvariante ist $R^1$ ein schwefelhaltiger Rest, etwa eine Sulfid-Gruppe und $R^2$ ist ein Alkan, Alken oder Alkin, wobei das Alkan, Alken oder Alkin als Option einen oder mehrere Substituenten aufweisen kann und eine Länge von maximal 40 Atomen, bevorzugt 6 bis 12 Atome, besonders bevorzugt 6 bis 10 Atome, entlang seiner längsten Kette aufweist. $R^2$ ist insofern bevorzugt ein Rest entsprechend $(Y)_n$. Ganz besonders bevorzugt ist es insofern, wenn $R^1$-$R^2$ ein Alkanthiol, Alkenthiol oder Alkinthiol, optional ein substituiertes Alkanthiol, Alkenthiol oder Alkinthiol, mit einer entsprechenden Kettenlänge ist. Solche Thiolverbindungen (Thioalkohole) können allgemein auch als Mercaptanverbindungen bezeichnet werden. Mit anderen Worten, in einer besonders bevorzugten Ausführungsform entsprechen die Ausgangsmoleküle der SAM beispielsweise der Formel

$$HS\text{-}(Y)_n\text{-}X$$

Bei allen diesen denkbaren Varianten für $R^1$ und $R^2$ bzw. $R^1$-$R^2$ ist es günstig, wenn das delokalisierte $\pi$-System der erfindungsgemäßen reaktive Gruppe X aus der Gruppe enthaltend konjugierte $\pi$-Systeme mit Kohlenstoffatomen als Bindungszentren, cyclisch konjugierte $\pi$-Systeme, $\pi$-Systeme von Resten mit mehreren cyclisch konjugierten $\pi$-Systemen ausgewählt sind. Dabei sind die cyclischen konjugierten $\pi$-Systeme bei Resten, die mehrere solcher Systeme aufweisen, über wenigstens einen Linker verbunden. Die cyclisch konjugierten $\pi$-Systeme sind bevorzugt planare cyclische Verbindungen, d.h. also planare cyclisch konjugierte $\pi$-Systeme. Ganz besonders bevorzugt sind die planaren cyclischen konjugierten $\pi$-Systeme aromatische oder heteroaromatische Systeme. Ist die reaktive Gruppe X ein Rest,

der mehrere solcher cyclischen planaren π-Systeme aufweist, welche über einen Linker verbunden sind, so ist der Linker bevorzugt ausgewählt aus einer Gruppe enthaltend C, Azogruppe, lineares konjugiertes π-System oder ein metallisches Zentralion, welches die cyclischen konjugierten π-Systeme als Metall-Komplex koordiniert. Günstig ist es dabei auch, wenn die gesamte Gruppe X eine planare Gruppe ist. In jedem Fall kann X sowohl elektronenziehende als auch elektronenschiebende Substituenten aufweisen. In einer bevorzugten Ausführungsvariante ist X ein aromatischer oder heteroaromatischer Rest oder ein Rest mit wenigstens einer aromatischen oder heteroaromatischen Gruppe. Dabei ist insbesondere auch denkbar, dass X ein substituierter aromatischer oder heteroaromatischer Rest ist.

Beispielsweise ist es vorstellbar, dass X ein aromatischer oder heteroaromatischer Rest mit wenigstens einem elektronenziehenden Substituenten ist. Ein entsprechender elektronenziehender Substituent kann bevorzugt ausgewählt sein aus der Gruppe enthaltend $COOR^4$, COOH, CHO, $C=(O)R^4$, CN, CH=CH-COOH, $NO_2$, $SO_3H$, $CF_3$, besonders bevorzugt aus der Gruppe enthaltend $CF_3$, CN, $NO_2$, wobei $R^4$ ausgewählt ist aus der Gruppe enthaltend H, Aryl, Alkyl, Heteroaryl, Heteroalkyl.

Denkbar ist auch, dass X ein aromatischer oder heteroaromatischer Rest mit wenigstens einem elektronenschiebenden Substituenten ist. Ein entsprechender elektronenschiebender Substituent kann bevorzugt ausgewählt sein aus der Gruppe enthaltend $NH_2$, $NR^5{}_2$, $OCH_3$, $CH_3$, OH, OR, $NHC=(O)R^5$, $OC=(O)R^5$, Aryl, Br, Cl, I, F, besonders bevorzugt ausgewählt aus der Gruppe enthaltend $CH_3$, $OCH_3$, $NH_2$, wobei $R^5$ ausgewählt ist aus der Gruppe enthaltend H, Aryl, Alkyl, Heteroaryl, Heteroalkyl, Halogenid.

Ein erfindungsgemäß aromatischer oder heteroaromatischer Rest kann beispielsweise ausgewählt sein aus der Gruppe enthaltend Furan, Pyrrol, Thiophen, Imidazol, Pyrazol, Oxyzol, Isoxazol, Thiazol, Benzofuran, Isobenzofuran, Indol, Isoindol, Benzothiophen, Benzimidazol, Purin, Indazol, Benzoxazol, Benzisoxazol, Benzothiazol, Benzol, Pyridin, Pyrazin, Pyrimidin, Pyridazin, Naphtalin, Anthracen, Chinolin, Isochinolin, Chinoxalin, Acridin, Chinazolin, Cinnolin und dergleichen. Jeder dieser Reste kann einen oder mehrere der oben beschriebenen elektronenziehenden oder elektronenschiebenden Substituenten aufweisen.

Ist das π-System des Rests X ein lineares, konjugiertes π-System, so ist beispielsweise denkbar, dass X ein Polymethin-Rest oder ein Carbonyl-Rest ist.

In einer besonders einfachen Ausführungsform kann X beispielsweise ein Phenyl-Rest sein. Vorstellbar ist auch, dass X ein Anthracen, Naphtalin, Furan, Indol, Pyridin, Pyrimidin oder Pyrrol-Rest ist. Alternativ ist auch denkbar, dass X ein substituierter Phenyl-Rest ist, wobei der Substituent ausgewählt ist aus der Gruppe enthaltend COOR4, COOH, CHO, $C=(O)R^4$, CN, CH=CH-COOH, $NO_2$, $SO_3H$, $CF_3$, besonders bevorzugt aus der Gruppe enthaltend $CF_3$, CN, $NO_2$, wobei $R^4$ ausgewählt ist aus der Gruppe enthaltend H, Aryl, Alkyl, Heteroaryl, Heteroalkyl. Besonders günstig ist dies, wenn $R^1$ eine Thiol-Gruppe und $R^2$ ein Rest entsprechend $(Y)_n$ mit $n \in \{0, ..., 40\}_z$, bevorzugt $n \in \{5, ..., 15\}_z$, besonders bevorzugt $n \in \{6, ..., 410\}_z$ ist. Die Moleküle der SAM können mithin der Formel

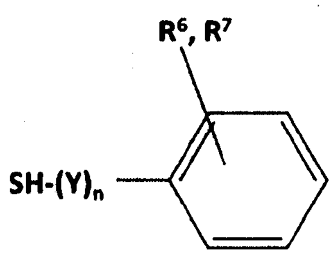

entsprechen.

$R^6$ ist dabei bevorzugt Wasserstoff oder ein oder mehrere elektronenschiebende Reste aus der Gruppe enthaltend $NH_2$, $NR^5{}_2$, $OCH_3$, $CH_3$, OH, OR, $NHC=(O)R^5$, $OC=(O)R^5$, Aryl, Br, Cl, I, F, besonders bevorzugt ausgewählt aus der Gruppe enthaltend $CH_3$, $OCH_3$, wobei $R^5$ ausgewählt ist aus der Gruppe enthaltend H, Aryl, Alkyl, Heteroaryl, Heteroalkyl, Halogenid.

$R^7$ ist bevorzugt Wasserstoff oder ein elektronenziehender Rest aus der Gruppe enthaltend $COOR^4$, COOH, CHO, $COR^4$, CN, CH=CH-COOH, $NO_2$, $SO_3H$, $CF_3$, besonders bevorzugt aus der Gruppe enthaltend $CF_3$, CN, $NO_2$, wobei $R^4$ ausgewählt ist aus der Gruppe enthaltend H, Aryl, Alkyl, Heteroaryl, Heteroalkyl.

$(Y)_n$ ist bevorzugt wie oben definiert, wobei für n bevorzugt $n \in \{0, ..., 40\}_z$, besonders bevorzugt $n \in \{5, ..., 15\}_z$, ganz besonders $n \in \{6, ..., 10\}_z$ gilt. Vorteilhaft ist es dabei, wenn Y ausgewählt ist aus der Gruppe enthaltend Alkan, Alken, Alkin, substituiertes Alkan, substituiertes Alken, substituiertes Alkin, Ether oder Amin, wobei die Substituenten ausgewählt sind aus der Gruppe Wasserstoff, Alkyl oder Aromat, besonders bevorzugt Wasserstoff oder Alkyl-Gruppen. Mithin entspricht eine besonders bevorzugte Ausführungsform der SAM-Moleküle der Formel

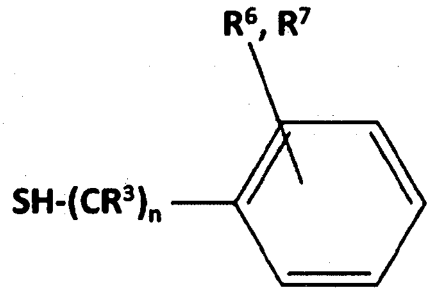

In einer weiteren Ausführungsform ist denkbar, dass X ein Polymethin-Rest ist, also ein konjugiertes Polyen, bei welchem ein Elektronenakzeptor über eine ungeradzahlige Kette von Methin-Gruppen mit einem Elektronen-Donor verknüpft ist. Beispielsweise kann X eine Gruppe entsprechend der Formel

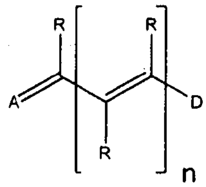

sein, wobei A ein Elektronenakzeptor, D ein Elektronendonor, R Wasserstoff oder Alkyl und n eine ganze Zahl oder Null ist. Dabei ist X bevorzugt über einen der Reste R mit dem Spacer $R^2$ kovalent verbunden. Besonders günstig ist dies, wenn $R^1$ eine Thiol-Gruppe und $R^2$ ein Rest entsprechend $(Y)_n$ mit $n \in \{0, ..., 40\}_Z$, bevorzugt $n \in \{5, ..., 15\}_Z$, besonders bevorzugt $n \in \{6, ..., 10\}_Z$ ist. Die Moleküle der SAM können mithin der Formel

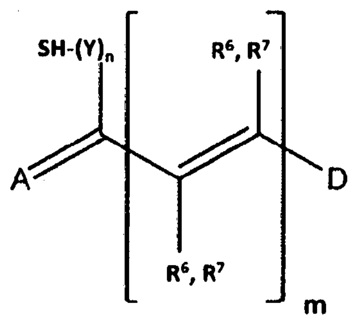

entsprechen. $R^6$ und D sind dabei bevorzugt Wasserstoff, Alkyl oder ein elektronenschiebender Rest aus der Gruppe enthaltend $NR^5_2$, $OCH_3$, $CH_3$, , $OR^5$, $NHC{=}(O)R^5$, $OC{=}(O)R^5$, Aryl, Br, Cl, I, F, besonders bevorzugt ausgewählt aus der Gruppe enthaltend $CH_3$, $OCH_3$, $NH_2$, wobei $R^5$ ausgewählt ist aus der Gruppe enthaltend H, Aryl, Alkyl, Heteroaryl, Heteroalkyl, Halogenid.

$R^7$ und A sind bevorzugt Wasserstoff, Alkyl oder ein elektronenziehender Rest aus der Gruppe enthaltend $COOR^4$, COOH, CHO, C=O, CN, CH=CH-COOH, $NO_2$, $SO_3H$, $CF_3$, besonders bevorzugt aus der Gruppe enthaltend $CF_3$, CN, $NO_2$, wobei $R^4$ ausgewählt ist aus der Gruppe enthaltend H, Aryl, Alkyl, Heteroaryl, Heteroalkyl.

Besonders bevorzugt sind $R^6$ und $R^7$ unabhängig voneinander entweder Wasserstoff oder ein Alkyl-Rest.

m ist eine ganze Zahl oder Null.

$(Y)_n$ ist bevorzugt wie oben definiert, wobei für n bevorzugt $n \in \{0, ..., 20\}_Z$, besonders bevorzugt $n \in \{5, ..., 15\}_Z$, ganz besonders bevorzugt $n \in \{6, ..., 10\}_Z$ gilt. Vorteilhaft ist es dabei, wenn Y ausgewählt ist aus der Gruppe enthaltend Alkan, Alken, Alkin, substituiertes Alkan, substituiertes Alken, substituiertes Alkin, Ether oder Amin, wobei die Substituenten ausgewählt sind aus der Gruppe Wasserstoff, Alkin oder Aryl, besonders bevorzugt Wasserstoff oder Alkyl. Mithin entspricht eine besonders bevorzugte Ausführungsform der SAM-Moleküle der Formel

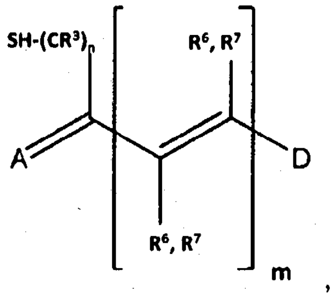

wobei $R^3$, $R^6$ und $R^7$ wie oben definiert sind.

In einer weiteren Ausführungsform kann X der Farbstoffgrundkörper eines Nitrofarbstoffes sein, also ein aromatischer Ring, an welchen mindestens eine Nitrogruppe gebunden ist. Beispielsweise kann X eine Gruppe entsprechend der Formel

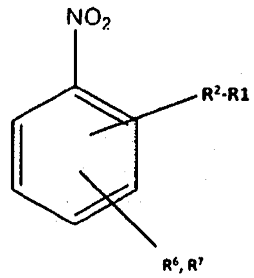

sein, wobei $R^6$, $R^7$ Wasserstoff oder ein stickstoffhaltiger organischer Rest, der über ein Stickstoffatom mit dem Nitrobenzylrest verbunden ist, sein kann und wobei X bevorzugt über diesen Rest $R^6$ oder $R^7$ oder ein weiteres Kohlenstoffatom des Ringes mit dem Spacer $R^2$ kovalent verbunden ist. Besonders günstig ist dies, wenn $R^1$ eine Thiol-Gruppe und $R^2$ ein Rest entsprechend $(Y)_n$ mit $n \in \{0, ..., 40\}_Z$, bevorzugt $n \in \{5, ..., 15\}_Z$, besonders $n \in \{6, ..., 10\}_Z$ ist. Die Moleküle der SAM können mithin der Formel

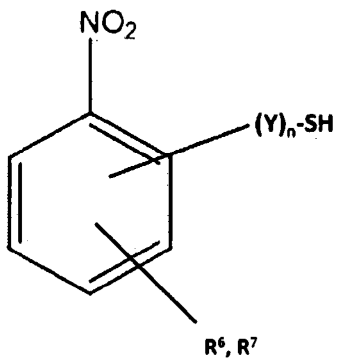

entsprechen. $R^6$ ist dabei bevorzugt Wasserstoff oder ein elektronenschiebender Rest aus der Gruppe enthaltend $NR^5_2$, $OCH_3$, $CH_3$, OH, OR, NHC=(O)$R^5$, OC=(O)$R^5$, Aryl, Br, Cl, I, F, besonders bevorzugt ausgewählt aus der Gruppe enthaltend $CH_3$, $OCH_3$, $NH_2$, wobei $R^5$ ausgewählt ist aus der Gruppe enthaltend H, Aryl, Alkyl, Heteroaryl, Heteroalkyl, Halogenid.

$R^7$ ist bevorzugt Wasserstoff oder ein elektronenziehender Rest aus der Gruppe enthaltend enthaltend $COOR^4$, COOH, CHO, C(O)$R^4$, CN, CH=CH-COOH, $NO_2$, $SO_3H$, $CF_3$, besonders bevorzugt aus der Gruppe enthaltend $CF_3$, CN, $NO_2$, wobei $R^4$ ausgewählt ist aus der Gruppe enthaltend H, Aryl, Alkyl, Heteroaryl, Heteroalkyl.

Besonders bevorzugt sind $R^6$ und $R^7$ unabhängig voneinander entweder Wasserstoff oder ein Rest entsprechend $NR^5R^4$, $NO_2$, NHC=(O)$R^5$, $NR^5_2$ oder $NH_2$, mit $R^5$, $R^4$ wie oben definiert.

$(Y)_n$ ist bevorzugt wie oben definiert, wobei für n bevorzugt $n \in \{0, ..., 40\}_Z$, besonders bevorzugt $n \in \{5, ..., 15\}_Z$, ganz besonders bevorzugt $n \in \{6, ..., 10\}_Z$ gilt. Vorteilhaft ist es dabei, wenn Y ausgewählt ist aus der Gruppe

enthaltend Alkan, Alken, Alkin, substituiertes Alkan, substituiertes Alken, substituiertes Alkin, Ether oder Amin, wobei die Substituenten ausgewählt sind aus der Gruppe Wasserstoff, Alkyl oder Aryl , besonders bevorzugt Wasserstoff oder Alkyl. Mithin entspricht eine besonders bevorzugte Ausführungsform der SAM-Moleküle der Formel

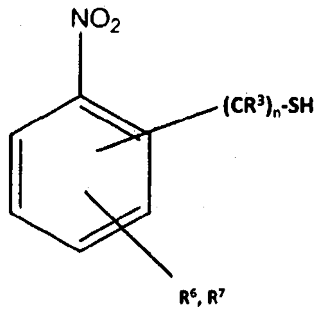

In noch einer weiteren Ausführungsform ist auch vorstellbar, dass X der Farbstoffgrundkörper eines Azofarbstoffes ist, bei welchem mindestens zwei aromatische Ringe über eine Azogruppe verknüpft sind. Beispielsweise kann X eine Gruppe entsprechend der Formel

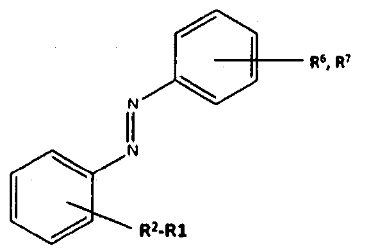

sein, wobei $R^6$ und $R^7$ jeweils unabhängig voneinander Wasserstoff, Aryl, Alky oder ein stickstoffhaltiger organischer Rest sein können. Besonders günstig ist dies, wenn $R^1$ eine Thiol-Gruppe und $R^2$ ein Rest entsprechend $(Y)_n$ mit $n \in \{0, ..., 40\}_z$, bevorzugt $n \in \{5, ..., 15\}_z$, besonders bevorzugt $n \in \{6, ..., 10\}_z$ ist. Die Moleküle der SAM können mithin der Formel

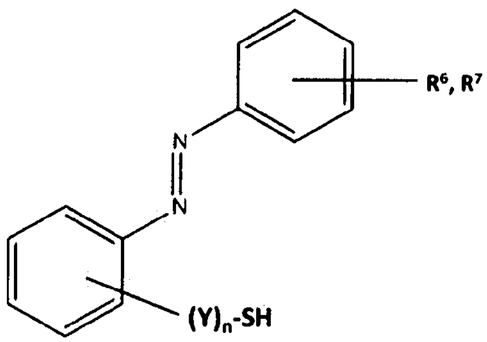

entsprechen. $R^6$ ist dabei bevorzugt Wasserstoff oder ein elektronenschiebender Rest aus der Gruppe enthaltend $NR^5_2$, $OCH_3$, $CH_3$,OH, OR, NHC=(O)$R^5$, OC=(O)$R^5$, Aryl, Br, Cl, I, F, besonders bevorzugt ausgewählt aus der Gruppe enthaltend $CH_3$, $OCH_3$, $NH_2$, wobei $R^5$ ausgewählt ist aus der Gruppe enthaltend H, Aryl, Alkyl, Heteroaryl, Heteroalkyl, Halogenid.

$R^7$ ist unabhängig von $R^6$ bevorzugt Wasserstoff oder ein elektronenziehender Rest aus der Gruppe enthaltend $COOR^4$, COOH, CHO, C(O)$R^4$, CN, CH=CH-COOH, $NO_2$, $SO_3H$, $CF_3$, besonders bevorzugt aus der Gruppe enthaltend $CF_3$, CN, $NO_2$, wobei $R^4$ ausgewählt ist aus der Gruppe enthaltend H, Aryl, Alkyl, Heteroaryl, Heteroalkyl.

Besonders bevorzugt sind $R^6$ und $R^7$ unabhängig voneinander entweder Wasserstoff, Aryl, Alkyl oder ein Rest entsprechend $NR^5R^4$, $NO_2$, NHC=(O)$R^5$, $NR^5_2$ oder $NH_2$, mit $R^5$, $R^4$ wie oben definiert.

$(Y)_n$ ist bevorzugt wie oben definiert, wobei für n bevorzugt $n \in \{0, ..., 40\}_z$, besonders bevorzugt $n \in \{5, ..., 15\}_z$, ganz besonders bevorzugt $n \in \{6, ..., 10\}_z$ gilt. Vorteilhaft ist es dabei, wenn Y ausgewählt ist aus der Gruppe enthaltend Alkan, Alken, Alkin, substituiertes Alkan, substituiertes Alken, substituiertes Alkin, Ether oder Amin wobei die Substituenten ausgewählt sind aus der Gruppe Wasserstoff, Alkyl und Aryl, besonders bevorzugt Wasserstoff und

Alkyl. Mithin entspricht eine besonders bevorzugte Ausführungsform der SAM-Moleküle der Formel

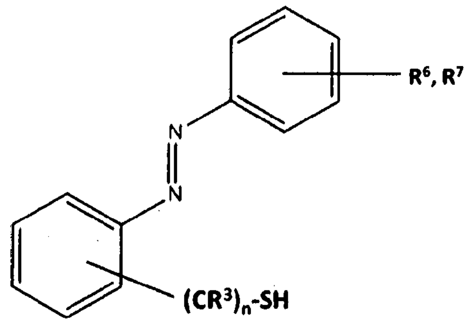

In einer weiteren Ausführungsform ist denkbar, dass die reaktive Gruppe X ein Farbstoffgrundkörper eines Carbonylfarbstoffes ist. Bei einem solchen Rest stehen mindestens zwei Carbonyl-Gruppen über eine oder mehrere $\pi$-Bindungen in Konjugation. Beispielsweise kann X eine Gruppe entsprechend der Formel

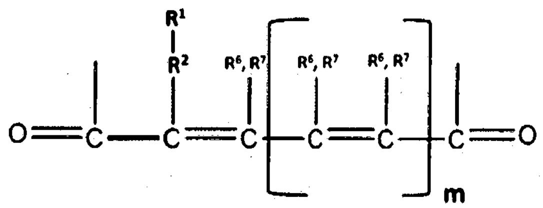

sein, wobei $R^6$ und $R^7$ jeweils unabhängig voneinander Wasserstoff, Aryl, Alkyl oder ein stickstoffhaltiger organischer Rest sein können. m ist eine ganze Zahl oder Null. Besonders günstig ist dies, wenn $R^1$ eine Thiol-Gruppe und $R^2$ ein Rest entsprechend $(Y)_n$ mit $n \in \{0, ..., 40\}_z$, bevorzugt $n \in \{5, ..., 15\}_z$, besonders bevorzugt $n \in \{6, ..., 10\}_z$ ist. Die Moleküle der SAM können mithin der Formel

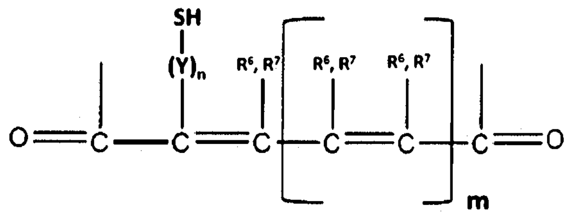

entsprechen. $R^6$ ist dabei bevorzugt Wasserstoff oder ein elektronenschiebender Rest aus der Gruppe enthaltend $NR^5_2$, $OCH_3$, $CH_3$, OH, OR, $NHC{=}(O)R^5$, $OC{=}(O)R^5$, Aryl, Br, Cl, I, F, besonders bevorzugt ausgewählt aus der Gruppe enthaltend $CH_3$, $OCH_3$, $NH_2$, wobei $R^5$ ausgewählt ist aus der Gruppe enthaltend H, Aryl, Alkyl, Heteroaryl, Heteroalkyl, Halogenid.

$R^7$ ist unabhängig von $R^6$ bevorzugt Wasserstoff oder ein elektronenziehender Rest aus der Gruppe enthaltend enthaltend $COOR^4$, COOH, CHO, $C(O)R^4$, CN, CH=CH-COOH, $NO_2$, $SO_3H$, $CF_3$, besonders bevorzugt aus der Gruppe enthaltend $CF_3$, CN, $NO_2$, wobei $R^4$ ausgewählt ist aus der Gruppe enthaltend H, Aryl, Alkyl, Heteroaryl, Heteroalkyl.

Besonders bevorzugt sind $R^6$ und $R^7$ unabhängig voneinander entweder Wasserstoff oder Aryl.

$(Y)_n$ ist bevorzugt wie oben definiert, wobei für n bevorzugt $n \in \{0, ..., 40\}_z$, besonders bevorzugt $n \in \{5, ..., 15\}_z$, ganz besonders bevorzugt $n \in \{6, ..., 10\}_z$ gilt. Vorteilhaft ist es dabei, wenn Y ausgewählt ist aus der Gruppe enthaltend Alkan, Alken, Alkin, substituiertes Alkan, substituiertes Alken, substituiertes Alkin, Ether oder Amin, wobei die Substituenten ausgewählt sind aus der Gruppe Wasserstoff, Alkyl oder Aryl, besonders bevorzugt Wasserstoff oder Alkyl-Gruppen enthält. Mithin entspricht eine besonders bevorzugte Ausführungsform der SAM-Moleküle der Formel

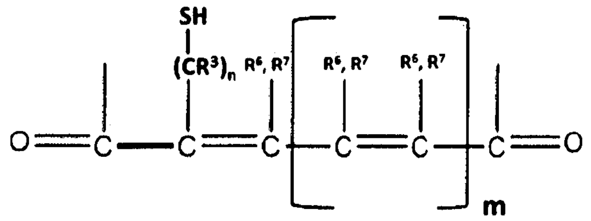

In einer weiteren Ausführungsform ist denkbar, dass die reaktive Gruppe X ein Farbstoffgrundkörper eines Triarylcarbenium-Farbstoffes ist. Solche Farbstoffgrundkörper sind Derivate des Triphenylmethans. Beispielsweise kann X eine Gruppe entsprechend der Formel

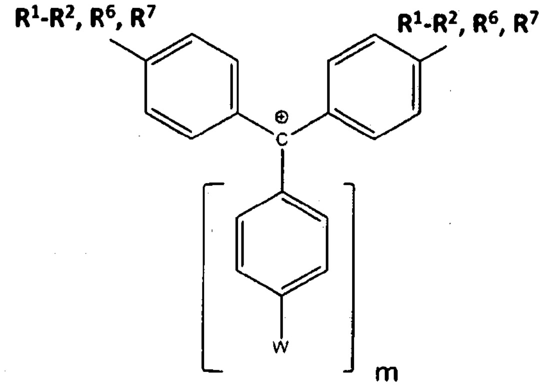

sein, wobei $R^6$ und $R^7$ jeweils unabhängig voneinander Wasserstoff, Aryl, Alkyl oder ein stickstoffhaltiger organischer Rest sein können. m ist ausgewählt aus 0 oder 1. W ist Wasserstoff oder ein stickstoffhaltiger organischer Rest. Besonders günstig ist dies, wenn $R^1$ eine Thiol-Gruppe und $R^2$ ein Rest entsprechend $(Y)_n$ mit $n \in \{0, ..., 40\}_Z$, bevorzugt $n \in \{5, ..., 15\}_Z$, besonders bevorzugt $n \in \{6, ..., 10\}_Z$ ist. Die Moleküle der SAM können mithin der Formel

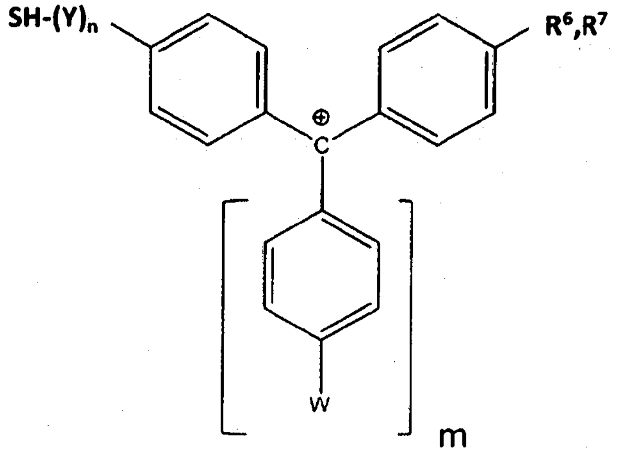

entsprechen. $R^6$ ist dabei bevorzugt Wasserstoff oder ein elektronenschiebender Rest aus der Gruppe enthaltend $NR^5_2$, $OCH_3$, $CH_3$, OH, OR, NHC=(O)$R^5$, OC=(O)$R^5$, Aryl, Br, Cl, I, F, besonders bevorzugt ausgewählt aus der Gruppe enthaltend $CH_3$, $OCH_3$, $NH_2$, wobei $R^5$ ausgewählt ist aus der Gruppe enthaltend H, Aryl, Alkyl, Heteroaryl, Heteroalkyl, Halogenid.

$R^7$ ist unabhängig von $R^6$ bevorzugt Wasserstoff oder ein elektronenziehender Rest aus der Gruppe enthaltend enthaltend COOR$^4$, COOH, CHO, C(O)$R^4$, CN, CH=CH-COOH, $NO_2$, $SO_3H$, $CF_3$, besonders bevorzugt aus der Gruppe enthaltend $CF_3$, CN, $NO_2$, wobei $R^4$ ausgewählt ist aus der Gruppe enthaltend H, Aryl, Alkyl, Heteroaryl, Heteroalkyl.

Besonders bevorzugt sind $R^6$ und $R^7$ unabhängig voneinander entweder ein stickstoffhaltiger organischer Rest gemäß der Formel $NR^4R^5$, Sauerstoff oder eine Hydroxylgruppe, mit $R^4$, $R^5$ wie oben definiert.

$(Y)_n$ ist bevorzugt wie oben definiert, wobei für n bevorzugt $n \in \{0, ..., 40\}_Z$, besonders bevorzugt $n \in \{5, ..., 15\}_Z$, ganz besonders bevorzugt $n \in \{6, ..., 10\}_Z$ gilt. Vorteilhaft ist es dabei, wenn Y ausgewählt ist aus der Gruppe

enthaltend Alkan, Alken, Alkin, substituiertes Alkan, substituiertes Alken, substituiertes Alkin, Ether oder Amin, wobei die Substituenten ausgewählt sind aus der Gruppe Wasserstoff, Alkyl oder Aryl, besonders bevorzugt Wasserstoff oder Alkyl enthält. Mithin entspricht eine besonders bevorzugte Ausführungsform der SAM-Moleküle der Formel

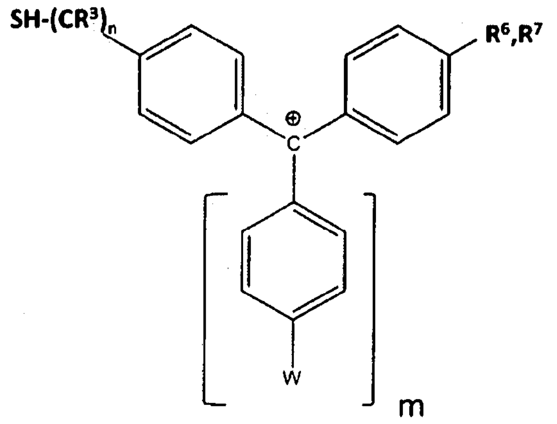

In einer weiteren Ausführungsform ist denkbar, dass die reaktive Gruppe X der Farbstoffgrundkörper eines Anthocyanidin-Farbstoffes ist. Solche Farbstoffgrundkörper sind hydroxylierte Derivate des Flavylium-Ions. Beispielsweise kann X eine Gruppe entsprechend der Formel

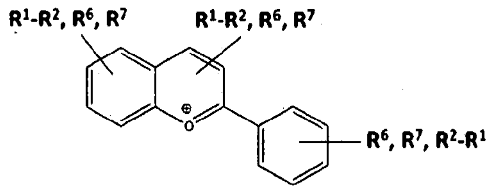

sein, wobei $R^6$ und $R^7$ jeweils unabhängig voneinander Wasserstoff oder ein Hydroxyl Rest sein können. Jedoch ist wenigstens ein Rest $R^6$ oder $R^7$ ein Hydroxyl-Rest. Besonders günstig ist dies, wenn $R^1$ eine Thiol-Gruppe und $R^2$ eine Rest entsprechend $(Y)_n$ mit $n \in \{0, ..., 40\}_z$, bevorzugt $n \in \{5, ..., 15\}_z$, besonders bevorzugt $n \in \{6, ..., 10\}_z$ ist. Die Moleküle der SAM können mithin einer der Formeln

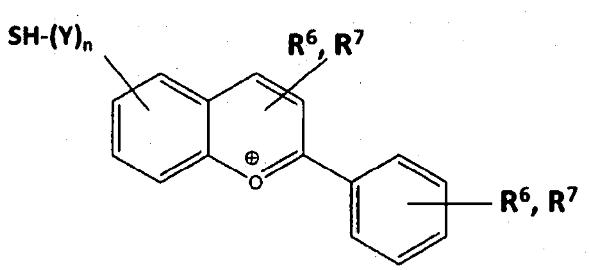

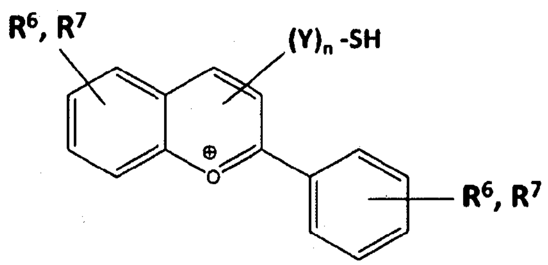

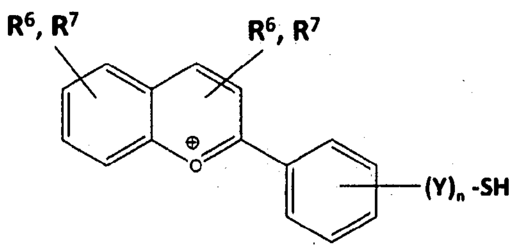

entsprechen. $R^6$ und $R^7$ sind dabei bevorzugt Wasserstoff oder ein elektronenschiebender Rest aus der Gruppe enthaltend $OCH_3$ oder OH, besonders bevorzugt ausgewählt aus der Gruppe enthaltend $OCH_3$ und OH.

Besonders bevorzugt sind $R^6$ und $R^7$ unabhängig voneinander entweder Wasserstoff oder ein Hydroxyl-Rest.

$(Y)_n$ ist bevorzugt wie oben definiert, wobei für n bevorzugt $n \in \{0, ..., 40\}_Z$, besonders bevorzugt $n \in \{5, ..., 15\}_Z$, ganz besonders bevorzugt $n \in \{6, ..., 10\}_Z$ gilt. Vorteilhaft ist es dabei, wenn Y ausgewählt ist aus der Gruppe enthaltend Alkan, Alken, Alkin, substituiertes Alkan, substituiertes Alken, substituiertes Alkin, Ether und Amin, wobei die Substituenten ausgewählt sind aus der Gruppe Wasserstoff, Alkyl und Aryl, besonders bevorzugt Wasserstoff und Alkyl. Mithin entspricht eine besonders bevorzugte Ausführungsform der SAM-Moleküle der Formel

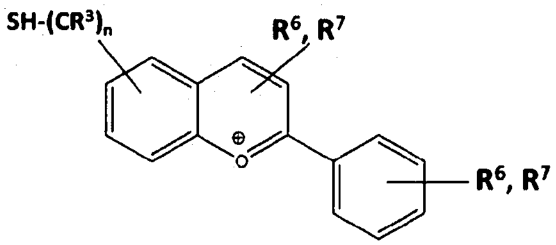

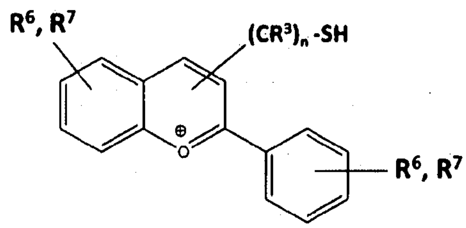

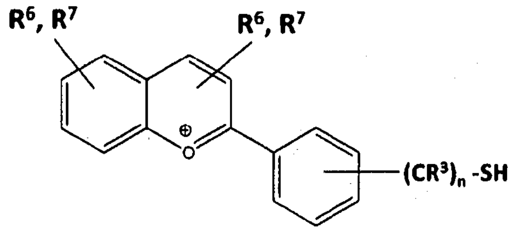

In einer weiteren Ausführungsform ist denkbar, dass die reaktive Gruppe X der Farbstoffgrundkörper eines Metallkomplex-Farbstoffes, etwa eines Phthalocyanin-Kupfer-Komplexes, ist. Beispielsweise kann X eine Gruppe entsprechend der Formel

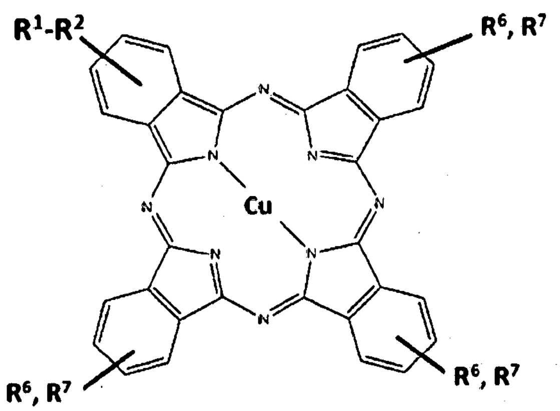

sein, wobei $R^6$ und $R^7$ jeweils unabhängig voneinander Wasserstoff oder Halogen sein können. Besonders günstig ist dies, wenn $R^1$ eine Thiol-Gruppe und $R^2$ ein Rest entsprechend $(Y)_n$ mit $n \in \{0, ..., 40\}_Z$, bevorzugt $n \in \{5, ..., 15\}_Z$, besonders bevorzugt $n \in \{6, ..., 10\}_Z$ ist. Die Moleküle der SAM können mithin der Formel

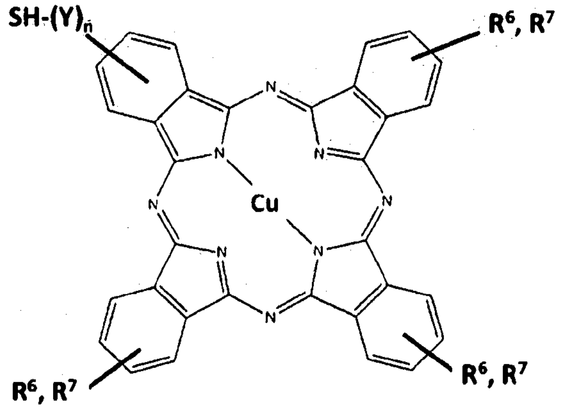

entsprechen. $R^6$ und $R^7$ sind ist dabei bevorzugt Wasserstoff oder Halogen.

Besonders bevorzugt sind $R^6$ und $R^7$ Wasserstoff.

$(Y)_n$ ist bevorzugt wie oben definiert, wobei für n bevorzugt $n \in \{0, ..., 40\}_Z$, besonders bevorzugt $n \in \{5, ..., 15\}_Z$, ganz besonders bevorzugt $n \in \{6, ..., 10\}_Z$ gilt. Vorteilhaft ist es dabei, wenn Y ausgewählt ist aus der Gruppe enthaltend Alkan, Alken, Alkin, substituiertes Alkan, substituiertes Alken, substituiertes Alkin, Ether oder Amin, wobei die Substituenten ausgewählt sind aus der Gruppe Wasserstoff, Alkyl oder Aryl, bevorzugt Wasserstoff und Alkyl, besonders bevorzugt Wasserstoff-Gruppen. Mithin entspricht eine besonders bevorzugte Ausführungsform der SAM-Moleküle der Formel

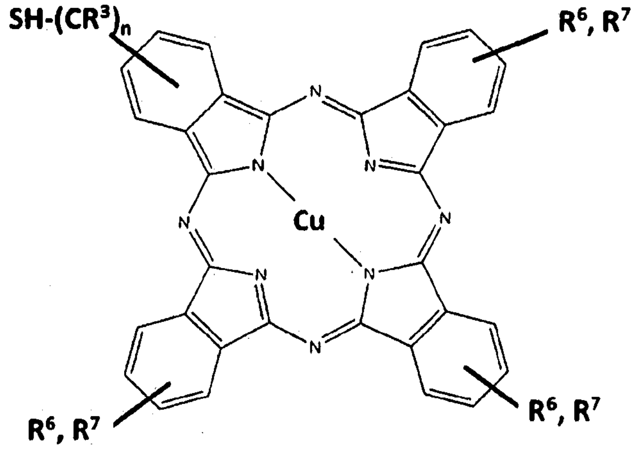

Mit anderen Worten X ist bevorzugt ausgewählt aus der Gruppe enthaltend Polyene; Nitrofarbstoffe; Azofarbstoffe; Triphenylmethanderivate; Anthocyanidine; Phthalocyanin-Metall-Komplexe. Alternativ ist auch vorstellbar, dass X ausgewählt ist aus der Gruppe enthaltend Aryl-Reste, die ausgewählt sind aus der Gruppe Phenyl, Benzyl, Pyridyl, Anthrachinone, Naphtalin. Dabei ist es insbesondere vorteilhaft, dass X ein Rest mit wenigstens einem elektronenziehenden Substituenten ist, wobei der Rest ausgewählt ist aus der Gruppe enthaltend Polymethin, Aryl-Reste, Metallkomplexe, makrocyclische Arenyl-Reste und Dendrimere, und wobei der Substituent bevorzugt ausgewählt ist aus der Gruppe enthaltend $COOR^4$, COOH, CHO, $COR^4$, CN, CH=CH-COOH, $NO_2$, $SO_3H$, $CF_3$, besonders bevorzugt aus der Gruppe enthaltend $CF_3$, CN, $NO_2$, wobei $R^4$ ausgewählt ist aus der Gruppe enthaltend H, Aryl, Alkyl, Heteroaryl, Heteroalkyl. Günstig kann es auch sein, dass X ein Rest mit wenigstens einem elektronenschiebenden Substituenten ist, wobei der Rest ausgewählt ist aus der Gruppe enthaltend Polymethin, Aryl-Reste, Metallkomplexe, makrocyclische Arenyl-Reste und Dendrimere, und wobei der Substituent bevorzugt ausgewählt ist aus der Gruppe enthaltend $NR^5_2$, $OCH_3$, $CH_3$, OH, OR, $NHC{=}(O)R^5$, $OC(O)R^5$, Aryl, Br, Cl, I, F, besonders bevorzugt ausgewählt aus der Gruppe enthaltend $CH_3$ und $OCH_3$, wobei $R^5$ ausgewählt ist aus der Gruppe enthaltend H, Aryl, Alkyl, Heteroaryl, Heteroalkyl, Halogenid.

In allen angeführten Ausführungsformen sind die Spacer $R^2$ benachbarter Moleküle kovalent miteinander verbunden.

In einer bevorzugten Ausführungsform der Erfindung ist die spannungsgesteuerte Auswerteeinrichtung ein Feldeffekttransistor, insbesondere ist der Feldeffekttransistor des erfindungsgemäßen Gassensors ein kapazitäts-kontrollierter Feldeffekttransistor (CCFET).

Vorteilhaft ist es dabei, wenn die Referenzelektrode mit der Gate-Elektrode des Feldeffekttransistors verbunden ist. Eine Änderung der Kapazität zwischen der Rezeptorschicht und der Referenzelektrode kann auf diese Weise eine Änderung des Ladungstransportes zwischen Source und Drain des FET bewirken. Diese Änderung des Stromflusses kann in letzter Konsequenz beispielsweise zur Auslösung eines Alarmes oder dergleichen genutzt werden.

In einem weiteren Aspekt sieht die Erfindung ein Gasmessgerät mit einem erfindungsgemäßen Gassensor vor. Außerdem sieht die Erfindung die Verwendung eines erfindungsgemäßen Gassensors zum Nachweis von volatilen organischen Verbindungen, bevorzugt Benzol und/oder Benzolderivaten vor.

Weitere Merkmale, Einzelheiten und Vorteile der Erfindung ergeben sich aus dem Wortlaut der Ansprüche, sowie aus der folgenden Beschreibung von Ausführungsbeispielen anhand der Zeichnungen. Es zeigen:

| | |
|---|---|
| Fig. 1 | eine schematische Darstellung eines erfindungsgemäßen Gassensors, |
| Fig. 2 | eine schematische Darstellung einer nicht erfindungsgemäßen Rezeptorschicht, |
| Fig. 3a bis 3h | verschiedene Beispiele für die reaktive Gruppe X der SAM-Moleküle, |
| Fig. 4 | Schematische Darstellung einer Analytbindung bei einem nicht erfindungsgemäßen Gassensor, |
| Fig. 5 | Nachweis von Benzol mit Hilfe eines nicht erfindungsgemäßen Gassensors. |

Der in Fig. 1 dargestellte erfindungsgemäße Gassensor 10 hat einen Messkanal 11, der von einem Deckel 14 bedeckt ist und einen Gaseinlass 12 sowie einen Gasauslass 13 aufweist. Das Volumen des Messkanales 11 wird von der Innenwand 111 begrenzt. Ein Messgasstrom M kann vom Gaseinlass 12 durch dieses Volumen hindurch zum Gasauslass 13 strömen und dabei an der Innenwand 111 entlang strömen.

Man erkennt in Fig. 1 außerdem, dass im Inneren des Messkanals 11 eine Rezeptorschicht 20 angeordnet ist. Die Rezeptorschicht 20 ist auf dem Deckel 14 aufgebracht. Sie besteht aus einem Träger 21 und einer Analytbindungsschicht 22.

Die Rezeptorschicht 20, insbesondere die Analytbindungsschicht 22, bildet dabei einen Teil der Innenwand 111 des Messkanales 11. Gegenüber der Rezeptorschicht 20 ist eine Referenzelektrode 30 angeordnet. Die Rezeptorschicht 20, die Referenzelektrode 30 und das zwischen der Rezeptorschicht 20 und der Referenzelektrode 30 ausgebildete Volumen des Messkanales 11 bilden einen Kondensator 40. Man erkennt insofern, dass die Referenzelektrode 30 kapazitiv mit der Rezeptorschicht 20 gekoppelt ist. Die Kapazität dieses Kondensators 40 ist durch Wechselwirkungen der Analytbindungsschicht 22 mit einem im Messgasstrom M enthaltenen Analyten veränderbar.

Man erkennt in Fig. 1 weiterhin, dass die Referenzelektrode 30 über eine elektrisch leitende Verbindung 31 mit einer spannungsgesteuerten Auswerteeinheit 50 verbunden ist. Die spannungsgesteuerte Auswerteeinheit 50 ist in dem gezeigten Beispiel ein Feldeffekttransistor, nämlich ein CCFET. Die Auswerteeinheit 50 weist insofern eine Source-Elektrode 51, eine Drain-Elektrode 52 und eine Gate-Elektrode 53 auf. Die Referenzelektrode 30 ist mit der Gate-Elektrode 53 verbunden. Die Gate-Elektrode 53 ist über einem Kanal 54 angeordnet, der die Source-Elektrode 51 und die Drain-Elektrode 52 miteinander verbindet. Die Gate-Elektrode 53 bestimmt in Abhängigkeit von der Kapazität des Kondensators 40 den Ladungsfluss, der zwischen Drain- und Source-Elektrode 51, 52 durch den Kanal 54 stattfindet.

Die Referenzelektrode 30 ist in dem gezeigten Ausführungsbeispiel in eine Isolationsschicht 60 eingebettet. Die Isolationsschicht 60 ist auf einem Substrat 61 angeordnet. Die Auswerteeinheit 50 ist zwischen der Isolationsschicht 60 und dem Substrat 61 angeordnet. Die Isolationsschicht 60 kann dabei helfen Fehlsignale zu vermeiden oder wenigstens zu minimieren.

Fig. 1 zeigt insofern einen Gassensor 10, wobei der Gassensor 10 einen Messkanal 11 mit einem Gaseinlass 12 und einem Gasauslass 13, mindestens eine Rezeptorschicht 20, eine Referenzelektrode 30 und eine spannungsgesteuerte Auswerteeinrichtung 50 aufweist, wobei die Referenzelektrode 30 kapazitiv mit der Rezeptorschicht 20 gekoppelt ist, wobei die Referenzelektrode 30 elektrisch leitend mit der Auswerteeinrichtung 50 verbunden ist, wobei die Rezeptorschicht 20 in dem Messkanal 11 ausgebildet ist, wobei der Messkanal 11 eine dielektrische Schicht zwischen der Rezeptorschicht 20 und der Referenzelektrode 30 bildet und wobei die Rezeptorschicht 20 einen Träger 21 und eine Analytbindungsschicht 22 aufweist.

Die Analytbindungsschicht 22 eines nicht erfindungsgemäßen Gassensors 10 ist - wie in Fig. 2 erkennbar - als selbstorganisierende monomolekulare Schicht (SAM) ausgebildet. Die Moleküle dieser SAM entsprechen der allgemeinen Formel $R^1$-$R^2$-X. Man erkennt, dass die Moleküle aus drei funktionellen Einheiten bestehen, nämlich der Kopplungsgruppe $R^1$, dem Spacer $R^2$ und der reaktiven Gruppe X. Die Moleküle der SAM, d.h. der Analytbindungsschicht 22, richten sich dabei auf dem Träger 21 in synchroner Orientierung und parallel zueinander aus. Dabei sind die Moleküle jeweils über die Kopplungsgruppe $R^1$ an den Träger 21 gekoppelt. Die Kopplungsgruppe $R^1$ ist dabei ausgewählt aus der Gruppe enthaltend Sulfid, Disulfid und Thiosulfat.

Man erkennt in Fig. 2, dass der Spacer $R^2$ den Abstand bestimmt, den die reaktive Gruppe X zur Kopplungsgruppe $R^1$ und mithin zum Träger 21 hat. Der Spacer $R^2$ ist dabei ausgewählt aus der Gruppe enthaltend Alkan, Alken, Alkin, Heteroalkan, Heteroalken, Heteroalkin, substituierte Alkane, substituierte Alkene, substituierte Alkine, substituierte

Heteroalkane, substituierte Heteroalkene, substituierte Heteroalkine.

Die reaktive Gruppe X ist eine organische oder metall-organische Gruppe. Zur Messung von Benzol besitzt die Gruppe X wenigstens ein delokalisiertes $\pi$-System. Der Träger 21 ist eine Schicht aus Metall, wobei das Metall ausgewählt ist aus der Gruppe enthaltend Gold, Platin, Palladium, Silber und Kupfer.

In den Figuren 3a bis 3h sind verschiedene Beispiele für Ausgestaltungen der reaktiven Gruppe X dargestellt. Es versteht sich von selbst, dass die Erfindung nicht auf die hier konkret dargestellten Moleküle beschränkt ist.

Entsprechend dem Ausführungsbeispiel von Fig. 3a ist die reaktive Gruppe X ein Phenylrest. Die reaktive Gruppe X ist dabei über eine kovalente Bindung zwischen dem Rest $R^2$ und einem der Ringatome des Phenylrestes mit dem Rest $R^2$-$R^1$ gekoppelt. $R^6$ und $R^7$ sind wie oben bereits beschrieben. In einem besonders günstigen (nicht in der Figur dargestellten) Ausführungsbeispiel mit einer solchen reaktiven Gruppe X entsprechen die Moleküle der SAM der Formel

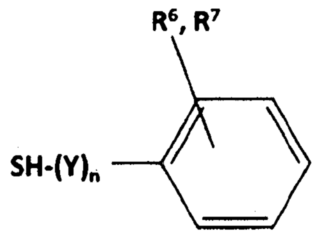

oder

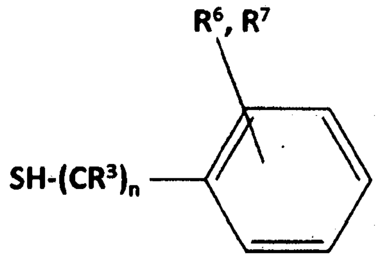

wobei auch hier $R^6$, $R^7$, $R^3$, n und Y wie oben beschrieben definiert sind.

Gemäß dem Ausführungsbeispiel von Fig. 3b ist die reaktive Gruppe X ein Nitrofarbstoff, bei welchem eine Nitrogruppe an einen aromatischen Ring gebunden ist, nämlich Nitrophenyl. Der aromatische Ring kann dabei weitere Substituenten gemäß der allgemeinen Formel $NR^4R^5$ aufweisen. Die reaktive Gruppe X ist dabei über den Ring an den Spacer $R^2$ gekoppelt. In einem besonders günstigen (nicht dargestellten) Ausführungsbeispiel mit einer solchen reaktiven Gruppe X entsprechen die Moleküle der SAM der Formel

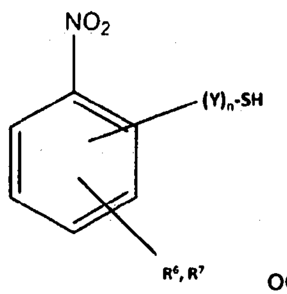

oder

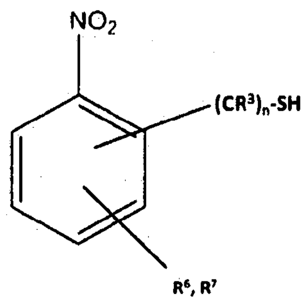

wobei auch hier $R^6$, $R^7$, $R^3$, n und Y wie oben beschrieben definiert sind.

In Fig. 3c ist die reaktive Gruppe X ein Azofarbstoff. Die reaktive Gruppe X ist dabei über einen der Ringe an den Spacer $R^2$ gekoppelt. In einem besonders günstigen (nicht dargestellten) Ausführungsbeispiel mit einer solchen reaktiven Gruppe X entsprechen die Moleküle der SAM der Formel

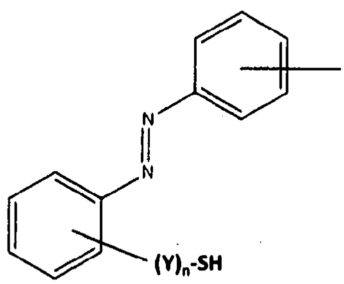

oder

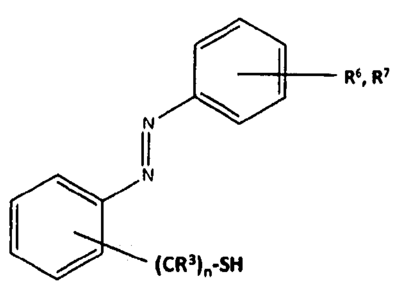

wobei $R^6$, $R^7$, $R^3$, n und Y wie oben beschrieben definiert sind.

Entsprechend dem Ausführungsbeispiel von Fig. 3d ist die reaktive Gruppe X ein Polymethinrest. Dabei kann

der Polymethinrest Alkylgruppen oder Wasserstoff als Substituenten $R^6$, $R^7$ aufweisen. Die reaktive Gruppe X ist dabei über einen solchen Rest an den Spacer $R^2$ gekoppelt. In einem besonders günstigen (in der Figur nicht dargestellten) Ausführungsbeispiel mit einer solchen reaktiven Gruppe X entsprechen die Moleküle der SAM der Formel

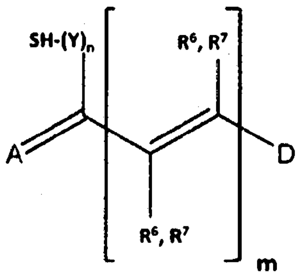

oder

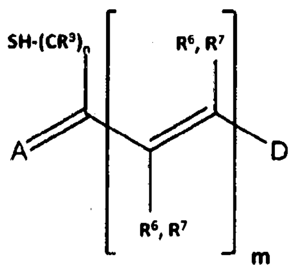

wobei $R^6$, $R^7$, $R^3$, n, m und Y wie oben beschrieben definiert sind.

Bei dem in Fig. 3e gezeigten Ausführungsbeispiel ist die reaktive Gruppe X ein ein Carbonylfarbstoff. Die reaktive Gruppe X ist dabei über einen Rest $R^6$ oder $R^7$ an den Spacer $R^2$ gekoppelt. In einem besonders günstigen (nicht dargestellten) Ausführungsbeispiel mit einer solchen reaktiven Gruppe X entsprechen die Moleküle der SAM der Formel

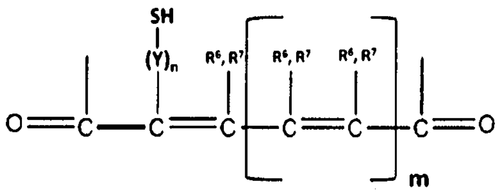

oder

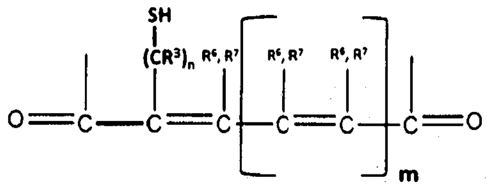

wobei $R^6$, $R^7$, $R^3$, n, m und Y wie oben beschrieben definiert sind.

Bei dem in Fig. 3f gezeigten Ausführungsbeispiel ist die reaktive Gruppe X ein Triarylcarbenium-Rest. Die reaktive Gruppe X ist dabei über einen der Ringe an den Spacer $R^2$ gekoppelt. In einem besonders günstigen (nicht dargestellten) Ausführungsbeispiel mit einer solchen reaktiven Gruppe X entsprechen die Moleküle der SAM der Formel

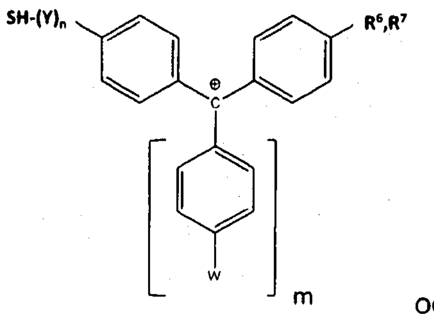

oder

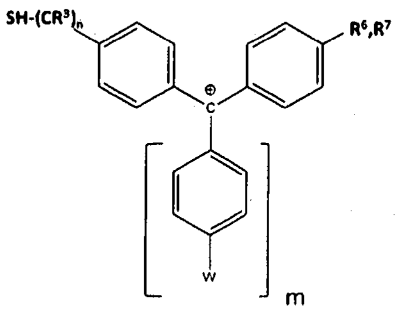

wobei $R^6$, $R^7$, $R^3$, n, m, W und Y wie oben beschrieben definiert sind.

In Fig. 3g ist die reaktive Gruppe ein Anthocyanderivat. Die reaktive Gruppe X ist dabei über einen der Ringe an den Spacer $R^2$ gekoppelt. In einem besonders günstigen (nicht dargestellten) Ausführungsbeispiel mit einer solchen reaktiven Gruppe X entsprechen die Moleküle der SAM einer der nachfolgenden Formeln

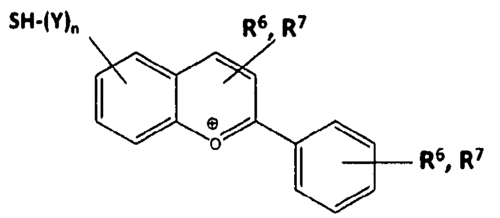

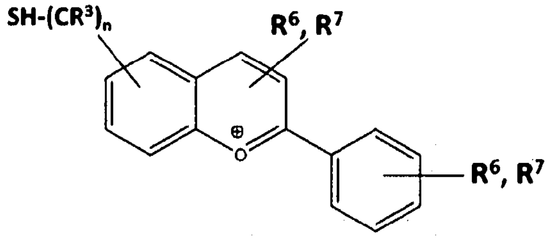

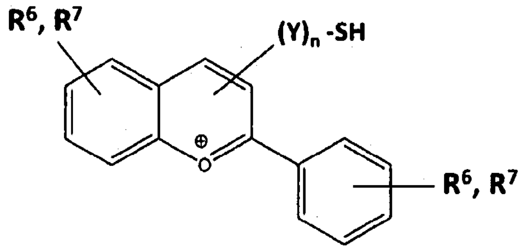

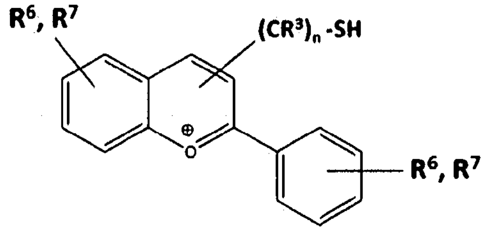

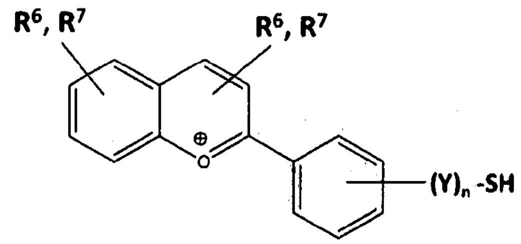

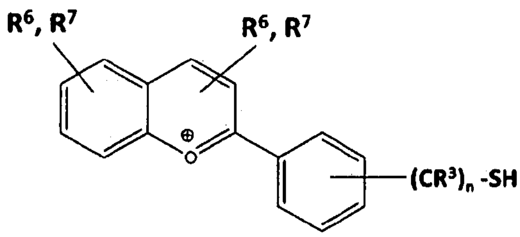

wobei $R^6$, $R^7$, $R^3$, n und Y wie oben beschrieben definiert sind.

In Fig. 3h ist die reaktive Gruppe ein Metallkomplex, nämlich Kupfer-Phthalocyanidin. Die reaktive Gruppe X ist dabei über einen der Ringe an den Spacer $R^2$ gekoppelt. In einem besonders günstigen (nicht dargestellten) Ausführungsbeispiel mit einer solchen reaktiven Gruppe X entsprechen die Moleküle der SAM der Formel

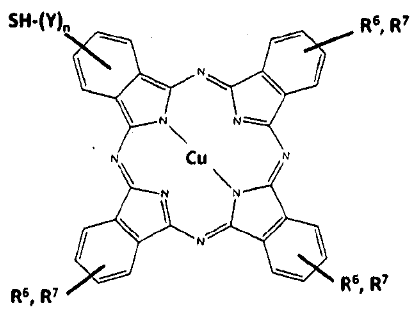

oder

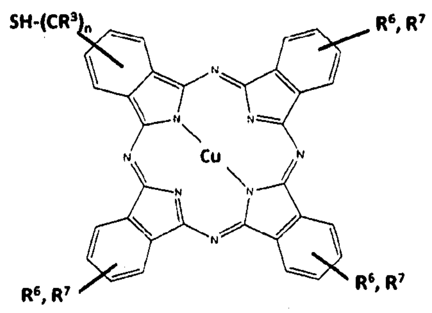

wobei $R^6$, $R^7$, $R^3$, n und Y wie oben beschrieben definiert sind.

In Fig. 4 erkennt man in einer nicht erfindungsgemäßen Darstellung, wie die sterische Anordnung der Moleküle der SAM, d.h. der Analytbindungsschicht 22, sein kann. Die Kopplung der Moleküle an den Träger 21 erfolgt über eine Schwefelbrücke. Die Kopplungsgruppe $R^1$ ist mithin eine Sulfid-Gruppe. Bei der Kopplung der Moleküle, d.h. bei der Ausbildung der Analytbindungsschicht 22 durch die Selbstorganisation der Moleküle, werden die Wasserstoff-Reste der Thiolgruppen abgespalten und es bildet sich eine kovalente Bindung zwischen den Schwefelatomen der Kopplungsgruppe $R^1$ und den Goldatomen des Trägers 21. Die Schwefelatome der Kopplungsgruppe $R^1$ sind außerdem kovalent mit dem Spacer $R^2$ verbunden. Der Spacer $R^2$ besteht in dem in Fig. 4 gezeigten nicht erfindungsgemäßen Beispiel aus einer linearen Kette von vier Methylengruppen. Es sei an dieser Stelle angemerkt, dass die Länge des Spacers $R^2$ bei besonders günstigen Ausführungsformen zwischen 6 und 12 Atomen beträgt. Allein aus Anschauungsgründen und zur besseren Darstellung ist in Fig. 4 die Länge des Spacers auf nur vier Methylengruppen reduziert. An die in Bezug auf die Kopplungsgruppe $R^1$ terminale Methylengruppe des Spacers $R^2$ ist die reaktive Gruppe X gebunden. Die reaktive Gruppe X ist in diesem Ausführungsbeispiel ein Phenylring. Der Phenylring trägt einen Substituenten $R^6$, $R^7$, nämlich eine NitroGruppe. Die SAM besteht mithin aus einer Schicht eines substituierten Phenylalkylmercaptans gemäß der

Formel

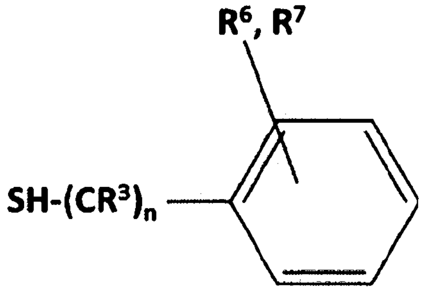

wobei $R^3$ Wasserstoff ist, n = 4, wobei die Ringatome des Phenylrestes in ortho und meta-Stellung jeweils Wasserstoff als Substituent $R^6$, $R^7$ tragen und wobei der aromatische Ring in para-Stelllung eine Nitrogruppe als Substituenten aufweist.

Man erkennt in der schematischen Darstellung in Fig. 4 weiterhin, wie ein Analyt A - im gezeigten Beispiel Benzol - sich mit dem Messgasstrom an die Analytbindungsschicht 22 annähern kann. Im nächsten Schritt können die Benzolmoleküle des Analyten A planparallel zu den Phenylresten der reaktiven Gruppe X der SAM addieren. Auf diese Weise kann es zu einer Ladungsverschiebung innerhalb der SAM kommen. Diese ist wiederum mit Hilfe der Auswerteeinrichtung wie oben beschrieben, nachweisbar.

In Fig. 5 erkennt man eine dynamische Meßkurve, die mit Hilfe eines Gassensors 10, welcher eine Analytbindungsschicht 20 entsprechend einem der oben dargestellten Beispiele aufweist, aufgenommen wurde. Insbesondere mit Hilfe der nicht erfindungsgemäßen Analytbindungsschicht 20, wie sie in Fig. 4 dargestellt ist, ist eine solche Messkurve erhältlich.

Man erkennt in Fig. 5 die Änderung der Austrittsarbeit in Abhängigkeit des Vorhandenseins eines Analyten. Dabei gibt die Kurve T die vom Gassensor 10 gemessene Austrittsarbeit wieder. Die Kurve B gibt die Konzentration eines Analyten A an, hier Benzol. Der Rezeptor wird zunächst mit benzolfreier Luft beaufschlagt. Nach 17 Sekunden wird eine Benzol-Konzentration von 1 ppm angelegt, wie man in der Kurve B erkennt. Man erkennt weiterhin, dass sich die Austrittsarbeit unmittelbar nach dem Zufügen von Benzol, nämlich nach ca. 3 Sekunden, um 5mV geändert hat. Nach 57 Sekunden wird der Benzolfluss unterbrochen. Die Austrittsarbeit geht innerhalb von 4 Sekunden auf den ursprünglichen Ausgangswert zurück. Nach 238 Sekunden wird die Benzolzufuhr wieder eingeschaltet und nach 264 Sekunden wieder ausgeschaltet. Man erkennt, dass auch bei dieser erneuten Beaufschlagung eine zuverlässige Änderung der Austrittsarbeit erfolgt.

Die Erfindung ist nicht auf eine der vorbeschriebenen Ausführungsformen beschränkt, sondern in vielfältiger Weise abwandelbar.

**Bezugszeichenliste**

| | | | |
|---|---|---|---|
| A | Analyt | 30 | Referenzelektrode |
| B | Analytkonzentration | 31 | Verbindung |
| M | Messgasstrom | | |
| $R^1$ | Kopplungsgruppe | 40 | Kondensator |
| $R^2$ | Spacer | | |
| T | Austrittsarbeit | 50 | Auswerteeinheit |
| X | reaktive Gruppe | 51 | Source-Elektrode |
| | | 52 | Drain-Elektrode |
| 10 | Gassensor | 53 | Gate-Elektrode |
| 11 | Messkanal | 54 | Kanal |
| 111 | Innenwand | | |
| 12 | Gaseinlass | 60 | Isolationsschicht |
| 13 | Gasauslass | 61 | Substrat |
| 14 | Deckel | | |
| 20 | Rezeptorschicht | | |
| 21 | Träger | | |
| 22 | Analytbindungsschicht | | |

## Patentansprüche

**1.** Gassensor (10) wobei der Gassensor (10) einen Messkanal (11) mit einem Gaseinlass (12) und einem Gasauslass (13), mindestens eine Rezeptorschicht (20), eine Referenzelektrode (30) und eine Auswerteeinrichtung (50) aufweist,

- wobei die Referenzelektrode (30) kapazitiv mit der Rezeptorschicht (20) gekoppelt ist,
- wobei die Referenzelektrode (30) elektrisch leitend mit der Auswerteeinrichtung (50) verbunden ist,
- wobei die Rezeptorschicht (20) in dem Messkanal (11) ausgebildet ist,
- wobei der Messkanal (11) eine dielektrische Schicht zwischen der Rezeptorschicht (20) und der Referenzelektrode (30) bildet, und
- wobei die Rezeptorschicht (20) einen Träger (21) und eine Analytbindungsschicht (22) aufweist
- wobei die Analytbindungsschicht (22) eine selbstorganisierende monomolekulare Schicht (SAM) ist, die aus Molekülen gemäß der allgemeinen Formel

$$\mathbf{R^1\text{-}R^2\text{-}X}$$

besteht,
- wobei $R^1$ eine Kopplungsgruppe ist, ausgewählt aus der Gruppe enthaltend Sulfid, Disulfid, Sulfinyl, Sulfino, Sulfo, Carbonothiol, Thiosulfat, Thiocyanat, Isothiocyanat, bevorzugt Sulfid oder Thiosulfat, und wobei die Moleküle der selbstorganisierenden monomolekularen Schicht jeweils über $R^1$ an den Träger gekoppelt sind;
- wobei der Träger eine Schicht aus Metall ist, wobei das Metall ausgewählt ist aus der Gruppe enthaltend Gold, Platin, Palladium, Silber und Kupfer;
- wobei $R^2$ ein Spacer ist, ausgewählt aus der Gruppe enthaltend Alkan, Alken, Alkin, Heteroalkan, Heteroalken, Heteroalkin, substituierte Alkane, substituierte Alkene, substituierte Alkine, substituierte Heteroalkane, substituierte Heteroalkene, substituierte Heteroalkine, Ether, Amine; und
- wobei X eine organische oder metall-organische Gruppe ist, die mit einem Analytmolekül in Wechselwirkung treten kann, insbesondere eine organische oder metall-organische Gruppe mit wenigstens einem delokalisierten $\pi$-System,
- und wobei die Spacer $R^2$ benachbarter Moleküle kovalent miteinander verbunden sind..

**2.** Gassensor nach Anspruch 1, **dadurch gekennzeichnet dass** der Träger (21) eine Schicht aus Gold ist.

**3.** Gassensor nach einem der Ansprüche 1 oder 2, **dadurch gekennzeichnet, dass** die Kopplungsgruppe $R^1$ kovalent mit dem Spacer $R^2$ und mit dem Träger (21) verbunden ist.

**4.** Gassensor nach einem der vorhergehenden Ansprüche, **dadurch gekennzeichnet, dass** die Kopplungsgruppe $R^1$ wenigstens eine Schwefelbrücke zwischen dem Spacer $R^2$ und dem Träger (21) ausbildet.

**5.** Gassensor nach einem der vorhergehenden Ansprüche, **dadurch gekennzeichnet, dass** $R^1$ ausgewählt ist aus der Gruppe enthaltend Sulfid, Disulfid oder Thiosulfat.

**6.** Gassensor nach einem der vorhergehenden Ansprüche, **dadurch gekennzeichnet, dass** $R^1$ ein Sulfid-Rest ist.

**7.** Gassensor nach einem der vorhergehenden Ansprüche, **dadurch gekennzeichnet, dass** $R^2$ ausgewählt ist aus der Gruppe enthaltend Alkane, Alkene, Alkine, substituierte Alkane, substituierte Alkene, substituierte Alkine, Ether, Amine, wobei die Substituenten der substituierten Alkane, Alkene oder Alkine ausgewählt sind aus der Gruppe enthaltend Wasserstoff, Alkyl oder Aryl.

**8.** Gassensor nach einem der vorhergehendend Ansprüche, **dadurch gekennzeichnet, dass** $R^2$ eine lineare Molekülgruppe entsprechend der Formel $(Y)_n$ mit $n \in \{0, ..., 40\}_z$ ist, wobei jedes Y unabhängig von den übrigen Y des jeweiligen $R^2$ ausgewählt ist aus der Gruppe enthaltend $CH_2$, CH, C, $CR^3$, O, N, $NR^3$, und wobei $R^3$ ausgewählt ist aus der Gruppe enthaltend H, Alkan, Alken, Alkin oder Aromat.

**9.** Gassensor nach Anspruch 8, **dadurch gekennzeichnet, dass** $n \in \{5, ..., 15\}_z$, bevorzugt $n \in \{6, ..., 10\}_z$ $n \in \{6, ..., 10\}_\mathbb{Z}$ ist.

**10.** Gassensor nach einem der vorhergehenden Ansprüche, **dadurch gekennzeichnet, dass** die Spacer $R^2$ benach-

barter Moleküle durch van-der Waals-Kräfte miteinander wechselwirken.

**11.** Gassensor nach einem der vorhergehenden Ansprüche, **dadurch gekennzeichnet, dass** das delokalisierte $\pi$-System der Gruppe X aus der Gruppe enthaltend konjugierte $\pi$-Systeme mit Kohlenstoffatomen als Bindungszentren, cyclisch konjugierte $\pi$-Systeme, $\pi$-Systeme von Resten mit mehreren cyclisch konjugierten $\pi$-Systemen ausgewählt ist.

**12.** Gassensor nach einem der vorhergehenden Ansprüche, **dadurch gekennzeichnet, dass** X ein aromatischer oder heteroaromatischer Rest mit wenigstens einem elektronenziehenden Substituenten ist.

**13.** Gassensor nach einem der vorhergehenden Ansprüche, **dadurch gekennzeichnet, dass** X ein aromatischer oder heteroaromatischer Rest mit wenigstens einem elektronenschiebenden Substituenten ist.

**14.** Gassensor nach einem der vorhergehenden Ansprüche, **dadurch gekennzeichnet, dass** X ausgewählt ist aus der Gruppe enthaltend Polyene; Nitrofarbstoffe; Azofarbstoffe; Triphenylmethanderivate; Anthocyanidine; Phthalocyanin-Metall-Komplexe.

**15.** Gassensor nach einem der vorhergehenden Ansprüche, **dadurch gekennzeichnet, dass** X ein Aryl-Rest ist, ausgewählt aus der Gruppe enthaltend Phenyl, Benzyl, Pyridyl, Anthrachinone, Naphtalin.

**16.** Gassensor nach einem der vorhergehenden Ansprüche, **dadurch gekennzeichnet, dass** X ein Rest mit wenigstens einem elektronenziehenden Substituenten ist, wobei der Rest ausgewählt ist aus der Gruppe enthaltend Polymethin, Aryl-Reste, Metallkomplexe, makrocyclische Arenyl-Reste und Dendrimere, und wobei der Substituent bevorzugt ausgewählt ist aus der Gruppe enthaltend $COOR^4$, COOH, CHO, $COR^4$, CN, CH=CH-COOH, $NO_2$, $SO_3H$, $CF_3$, besonders bevorzugt aus der Gruppe enthaltend $CF_3$, CN, $NO_2$, wobei $R^4$ ausgewählt ist aus der Gruppe enthaltend H, Aryl, Alkyl, Heteroaryl, Heteroalkyl.

**17.** Gassensor nach einem der vorhergehenden Ansprüche, **dadurch gekennzeichnet, dass** X ein Rest mit wenigstens einem elektronenschiebenden Substituenten ist, wobei der Rest ausgewählt ist aus der Gruppe enthaltend Polymethin, Aryl-Reste, Metallkomplexe, makrocyclische Arenyl-Reste und Dendrimere, und wobei der Substituent bevorzugt ausgewählt ist aus der Gruppe enthaltend , $NR^5_2$, $OCH_3$, $CH_3$, OH, OR, NHC=(O)$R^5$, OC(O)$R^5$, Aryl, Br, Cl, I, F, besonders bevorzugt ausgewählt aus der Gruppe enthaltend $CH_3$ und $OCH_3$, wobei $R^5$ ausgewählt ist aus der Gruppe enthaltend H, Aryl, Alkyl, Heteroaryl, Heteroalkyl, Halogenid.

**18.** Gassensor nach einem der vorhergehenden Ansprüche, **dadurch gekennzeichnet, dass** die Auswerteeinrichtung (50) ein Feldeffekttransistor ist, insbesondere ist der Feldeffekttransistor des erfindungsgemäßen Gassensors ein kapazitäts-kontrollierter Feldeffekttransistor (CCFET).

**19.** Gassensor nach einem der vorhergehenden Ansprüche, **dadurch gekennzeichnet, dass** die Referenzelektrode (30) mit der Gate-Elektrode (53) des Feldeffekttransistors verbunden ist.

**20.** Gasmessgerät mit einem Gassensor (10) gemäß einem der vorherigen Ansprüche.

**21.** Verwendung eines Gassensors (10) gemäß einem der vorhergehenden Ansprüche zum Nachweis von volatilen organischen Verbindungen, bevorzugt Benzol und/oder Benzolderivaten.

## Claims

**1.** A gas sensor (10), wherein the gas sensor (10) has a measuring channel (11) with a gas inlet (12) and a gas outlet (13), at least one receptor layer (20), a reference electrode (30) and an analysis device (50),

- wherein the reference electrode (30) is capacitively coupled with the receptor layer (20),
- wherein the reference electrode (30) is connected in an electively conductive manner to the analysis device (50),
- wherein the receptor layer (20) is formed in the measuring channel (11),
- wherein the measuring channel (11) forms a dielectric layer between the receptor layer (20) and the reference electrode (30), and
- wherein the receptor layer (20) has a support (21) and an analyte-binding layer (22),

• wherein the analyte-binding layer (22) is a self-assembling monolayer (SAM) which consists of molecules in accordance with the general formula

$$R^1-R^2-X,$$

• wherein $R^1$ is a coupling group, selected from the group containing sulphide, disulphide, sulphinyl, sulphino, sulpho, carbonothiol, thiosulphate, thiocyanate, isothiocyanate, preferably sulphide or thiosulphate, and wherein the molecules of the self-assembling monolayer are each coupled via $R^1$ to the support;
• wherein the support is a layer of metal, wherein the metal is selected from the group containing gold, platinum, palladium, silver and copper;
• wherein $R^2$ is a spacer, selected from the group containing alkane, alkene, alkyne, heteroalkane, heteroalkene, heteroalkyne, substituted alkanes, substituted alkenes, substituted alkynes, substituted heteroalkanes, substituted heteroalkenes, substituted heteroalkynes, ethers, amines; and
• wherein X is an organic or organometallic group, which can interact with an analyte molecule, in particular an organic or organometallic group with at least one delocalized $\pi$-system,
• and wherein the spacers $R^2$ of adjacent molecules are bound covalently to one another.

**2.** A gas sensor according to claim 1, **characterised in that** the support (21) is a layer of gold.

**3.** A gas sensor according to one of claims 1 or 2, **characterised in that** the coupling group $R^1$ is bound covalently to the spacer $R^2$ and to the support (21).

**4.** A gas sensor according to one of the preceding claims, **characterised in that** the coupling group $R^1$ forms at least one sulphur bridge between the spacer $R^2$ and the support (21).

**5.** A gas sensor according to one of the preceding claims, **characterised in that** $R^1$ is selected from the group containing sulphide, disulphide or thiosulphate.

**6.** A gas sensor according to one of the preceding claims, **characterised in that** $R^1$ is a sulphide radical.

**7.** A gas sensor according to one of the preceding claims, **characterised in that** $R^2$ is selected from the group containing alkanes, alkenes, alkynes, substituted alkanes, substituted alkenes, substituted alkynes, ethers, amines, wherein the substituents of the substituted alkanes, alkenes or alkynes are selected from the group containing hydrogen, alkyl or aryl.

**8.** A gas sensor according to one of the preceding claims, **characterised in that** $R^2$ is a linear molecular group corresponding to the formula $(Y)_n$ with $n \in \{0, \ldots, 40\}\mathbb{Z},$ wherein each Y is selected, independently from the other Y values of the respective $R^2$, from the group containing $CH_2$, CH, C, $CR^3$, O, N, $NR^3$, and wherein $R^3$ is selected from the group containing H, alkane, alkene, alkyne or an aromatic.

**9.** A gas sensor according to claim 8, **characterised in that** $n \in \{5, \ldots, 15\}\mathbb{Z},$ preferably $n \in \{6, \ldots, 10\}\mathbb{Z}.$

**10.** A gas sensor according to one of the preceding claims, **characterised in that** the spacers $R^2$ of adjacent molecules interact with one another by means of Van der Waals forces.

**11.** A gas sensor according to one of the preceding claims, **characterised in that** the delocalized $\pi$-system of group X is selected from the group containing conjugated $\pi$-systems with carbon atoms as binding centres, cyclically conjugated $\pi$-systems, $\pi$-systems of radicals with a plurality of cyclically conjugated $\pi$-systems.

**12.** A gas sensor according to one of the preceding claims, **characterised in that** X is an aromatic or heteroaromatic radical with at least one electron-drawing substituent.

**13.** A gas sensor according to one of the preceding claims, **characterised in that** X is an aromatic or heteroaromatic radical with at least one electron-pushing substituent.

**14.** A gas sensor according to one of the preceding claims, **characterised in that** X is selected from the group containing polyenes; nitro dyes; azo dyes; triphenylmethane derivatives; anthocyanidins; phthalocyanine-metal complexes.

**15.** A gas sensor according to one of the preceding claims, **characterised in that** X is an aryl radical, selected from the group containing phenyl, benzyl, pyridyl, antraquinones, naphthalene.

**16.** A gas sensor according to one of the preceding claims, **characterised in that** X is a radical with at least one electron-drawing substituent, wherein the radical is selected from the group containing polymethine, aryl radicals, metal complexes, macrocyclic arenyl radicals and dendrimers, and wherein the substituent is preferably selected from the group containing $COOR^4$, COOH, CHO, $COR^4$, CN, CH=CH-COOH, $NO_2$, $SO_3H$, $CF_3$, particularly preferably from the group containing $CF_3$, CN, $NO_2$, wherein $R^4$ is selected from the group containing H, aryl, alkyl, heteroaryl, heteroalkyl.

**17.** A gas sensor according to one of the preceding claims, **characterised in that** X is a radical with at least one electron-pushing substituent, wherein the radical is selected from the group containing polymethine, aryl radicals, metal complexes, macrocyclic arenyl radicals and dendrimers, and wherein the substituent is preferably selected from the group containing $NR^5_2$, $OCH_3$, $CH_3$, OH, OR, NHC=(O)$R^5$, OC(O)$R^5$, aryl, Br, Cl, I, F, particularly preferably selected from the group containing $CH_3$ and $OCH_3$, wherein $R^5$ is selected from the group containing H, aryl, alkyl, heteroaryl, heteroalkyl, halide.

**18.** A gas sensor according to one of the preceding claims, **characterised in that** the analysis device (50) is a field-effect transistor, the field-effect transistor of the gas sensor in accordance with the invention being in particular a capacitively controlled field-effect transistor (CCFET).

**19.** A gas sensor according to one of the preceding claims, **characterised in that** the reference electrode (30) is connected to the gate electrode (53) of the field-effect transistor.

**20.** A gas-measuring device having a gas sensor (10) in accordance with one of the previous claims.

**21.** Use of a gas sensor (10) in accordance with one of the preceding claims for detecting volatile organic compounds, preferably benzene and/or benzene derivatives.

## Revendications

**1.** Capteur de gaz (10), le capteur de gaz (10) présentant un canal de mesure (11) pourvu d'une entrée de gaz (12) et d'une sortie de gaz (13), au moins une couche réceptrice (20), une électrode de référence (30) et un dispositif d'évaluation (50),

- l'électrode de référence (30) étant couplée de manière capacitive à la couche réceptrice (20),
- l'électrode de référence (30) étant connectée de manière électriquement conductrice au dispositif d'évaluation (50),
- la couche réceptrice (20) étant réalisée dans le canal de mesure (11),
- le canal de mesure (11) formant une couche diélectrique entre la couche réceptrice (20) et l'électrode de référence (30) et
- la couche réceptrice (20) présentant un support (21) et une couche de liaison (22) d'analytes
- la couche de liaison (22) d'analytes étant une couche monomoléculaire auto-organisatrice (SAM) qui est constituée par des molécules de formule générale

$$R^1\text{-}R^2\text{-}X$$

  - $R^1$ étant un groupe de couplage, choisi dans le groupe contenant sulfure, disulfure, sulfinyle, sulfino, sulfo, carbonothiol, thiosulfate, thiocyanate, isothiocyanate, de préférence sulfure ou thiosulfate et les molécules de la couche monomoléculaire auto-organisatrice étant à chaque fois couplées au support via $R^1$ ;

- le support étant une couche métallique, le métal étant choisi dans le groupe contenant l'or, le platine, le palladium, l'argent et le cuivre ;

• $R^2$ étant un écarteur, choisi dans le groupe contenant alcane, alcène, alcyne, hétéroalcane, hétéroalcène, hétéroalcyne, les alcanes substitués, les alcènes substitués, les alcynes substituées, les hétéroalcanes substitués, les hétéroalcènes substitués, les hétéroalcynes substitués, les éthers, les amines ; et
• X étant un groupe organique ou organométallique, qui peut entrer en interaction avec une molécule d'analyte, en particulier un groupe organique ou organométallique présentant au moins un système $\pi$ délocalisé
• et les molécules adjacentes à l'écarteur $R^2$ étant liées par covalences les unes aux autres.

**2.** Capteur de gaz selon la revendication 1, **caractérisé en ce que** le support (21) est une couche d'or.

**3.** Capteur de gaz selon l'une quelconque des revendications 1 ou 2, **caractérisé en ce que** le groupe de couplage $R^1$ est lié par covalence à l'écarteur $R^2$ et au support (21).

**4.** Capteur de gaz selon l'une quelconque des revendications précédentes, **caractérisé en ce que** le groupe de couplage $R^1$ forme au moins un pont soufré entre l'écarteur $R^2$ et le support (21) .

**5.** Capteur de gaz selon l'une quelconque des revendications précédentes, **caractérisé en ce que** $R^1$ est choisi dans le groupe contenant sulfure, disulfure ou thiosulfate.

**6.** Capteur de gaz selon l'une quelconque des revendications précédentes, **caractérisé en ce que** $R^1$ est un radical sulfure.

**7.** Capteur de gaz selon l'une quelconque des revendications précédentes, **caractérisé en ce que** $R^2$ est choisi dans le groupe contenant les alcanes, les alcènes, les alcynes, les alcanes substitués, les alcènes substitués, les alcynes substitués, les éthers, les amines, les substituants des alcanes, des alcènes ou des alcynes substitués étant choisis dans le groupe contenant hydrogène, alkyle ou aryle.

**8.** Capteur de gaz selon l'une quelconque des revendications précédentes, **caractérisé en ce que** $R^2$ est un groupe moléculaire linéaire correspondant à la formule $(Y)_n$ dans laquelle $n \in \{0, ..., 40\}_Z$, chaque Y étant choisi, indépendamment des autres Y du radical $R^2$ respectif dans le groupe contenant $CH_2$, CH, C, $CR^3$, O, N, $NR^3$ et $R^3$ étant choisi dans le groupe contenant H, alcane, alcène, alcyne ou composé aromatique.

**9.** Capteur de gaz selon la revendication 8, **caractérisé en ce que** $n \in \{5, ..., 15\}_Z$, de préférence n e $\{6, ..., 10\}_Z$.

**10.** Capteur de gaz selon l'une quelconque des revendications précédentes, **caractérisé en ce que** les molécules adjacentes à l'écarteur $R^2$ interagissent les unes avec les autres par des forces de van der Waals.

**11.** Capteur de gaz selon l'une quelconque des revendications précédentes, **caractérisé en ce que** le système $\pi$ délocalisé du groupe X est choisi dans le groupe contenant les systèmes $\pi$ conjugués présentant des atomes de carbone comme centres de liaison, les systèmes $\pi$ cycliquement conjugués, les systèmes $\pi$ de radicaux présentant plusieurs systèmes $\pi$ cycliquement conjugués.

**12.** Capteur de gaz selon l'une quelconque des revendications précédentes, **caractérisé en ce que** X est un groupe aromatique ou hétéroaromatique présentant au moins un substituant attracteur d'électrons.

**13.** Capteur de gaz selon l'une quelconque des revendications précédentes, **caractérisé en ce que** X est un groupe aromatique ou hétéroaromatique présentant au moins un substituant déplaçant les électrons.

**14.** Capteur de gaz selon l'une quelconque des revendications précédentes, **caractérisé en ce que** X est choisi dans le groupe contenant les polyènes ; les colorants nitro ; les colorants azo ; les dérivés de triphénylméthane ; les anthocyanidines ; les complexes de type phtalocyanine-métal.

**15.** Capteur de gaz selon l'une quelconque des revendications précédentes, **caractérisé en ce que** X est un radical aryle, choisi dans le groupe contenant phényle, benzyle, pyridyle, anthraquinone, naphtalène.

**16.** Capteur de gaz selon l'une quelconque des revendications précédentes, **caractérisé en ce que** X est un radical présentant au moins un substituant attracteur d'électrons, le radical étant choisi dans le groupe contenant la polyméthine, les radicaux aryle, les complexes métalliques, les radicaux arényle macrocycliques et les dendrimères et le substituant étant de préférence choisi dans le groupe contenant $COOR^4$, COOH, CHO, $COR^4$, CN, CH=CH-

COOH, $NO_2$, $SO_3H$, $CF_3$, de manière particulièrement préférée dans le groupe contenant $CF_3$, CN, $NO_2$, $R^4$ étant choisi dans le groupe contenant H, aryle, alkyle, hétéroaryle, hétéroalkyle.

**17.** Capteur de gaz selon l'une quelconque des revendications précédentes, **caractérisé en ce que** X est un radical présentant au moins un substituant déplaçant les électrons, le radical étant choisi dans le groupe contenant la polyméthine, les radicaux aryle, les complexes métalliques, les radicaux arényle macrocycliques et les dendrimères et le substituant étant de préférence choisi dans le groupe contenant $NR^5_2$, $OCH_3$, $CH_3$, OH, OR, NHC=(O)$R^5$, OC(O)$R^5$, aryle, Br, Cl, I, F, de manière particulièrement préférée choisi dans le groupe contenant $CH_3$ et $OCH_3$, $R^5$ étant choisi dans le groupe contenant H, aryle, alkyle, hétéroaryle, hétéroalkyle, halogénure.

**18.** Capteur de gaz selon l'une quelconque des revendications précédentes, **caractérisé en ce que** le dispositif d'évaluation (50) est un transistor à effet de champ, en particulier, le transistor à effet de champ du capteur de gaz selon l'invention est un transistor à effet de champ à capacité contrôlée (CCFET).

**19.** Capteur de gaz selon l'une quelconque des revendications précédentes, **caractérisé en ce que** l'électrode de référence (30) est reliée à l'électrode grille (53) du transistor à effet de champ.

**20.** Appareil de mesure de gaz présentant un capteur de gaz (10) selon l'une quelconque des revendications précédentes.

**21.** Utilisation d'un capteur de gaz (10) selon l'une quelconque des revendications précédentes pour détecter des composés organiques volatils, de préférence le benzène et/ou les dérivés du benzène.

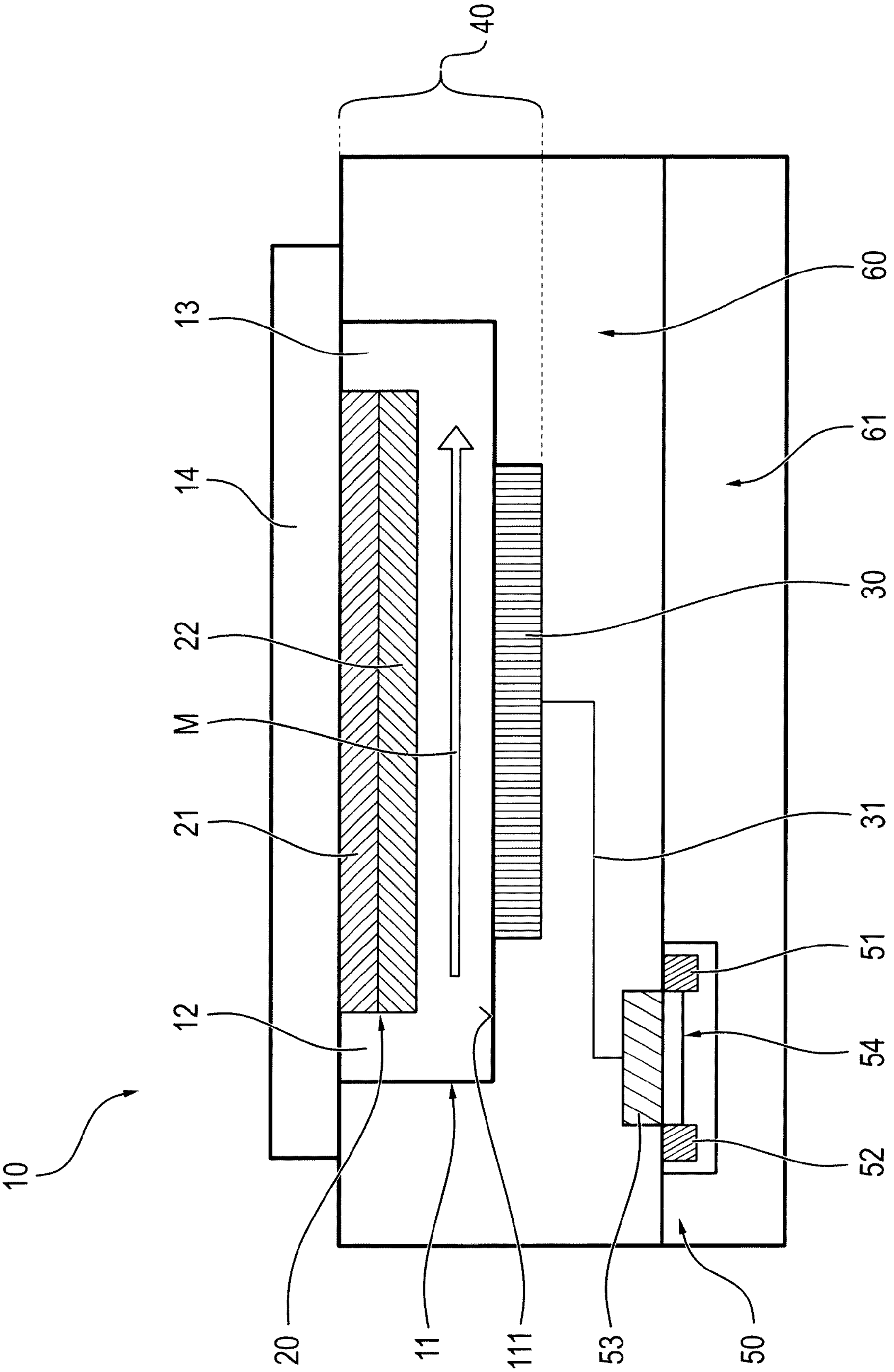


FIG. 1

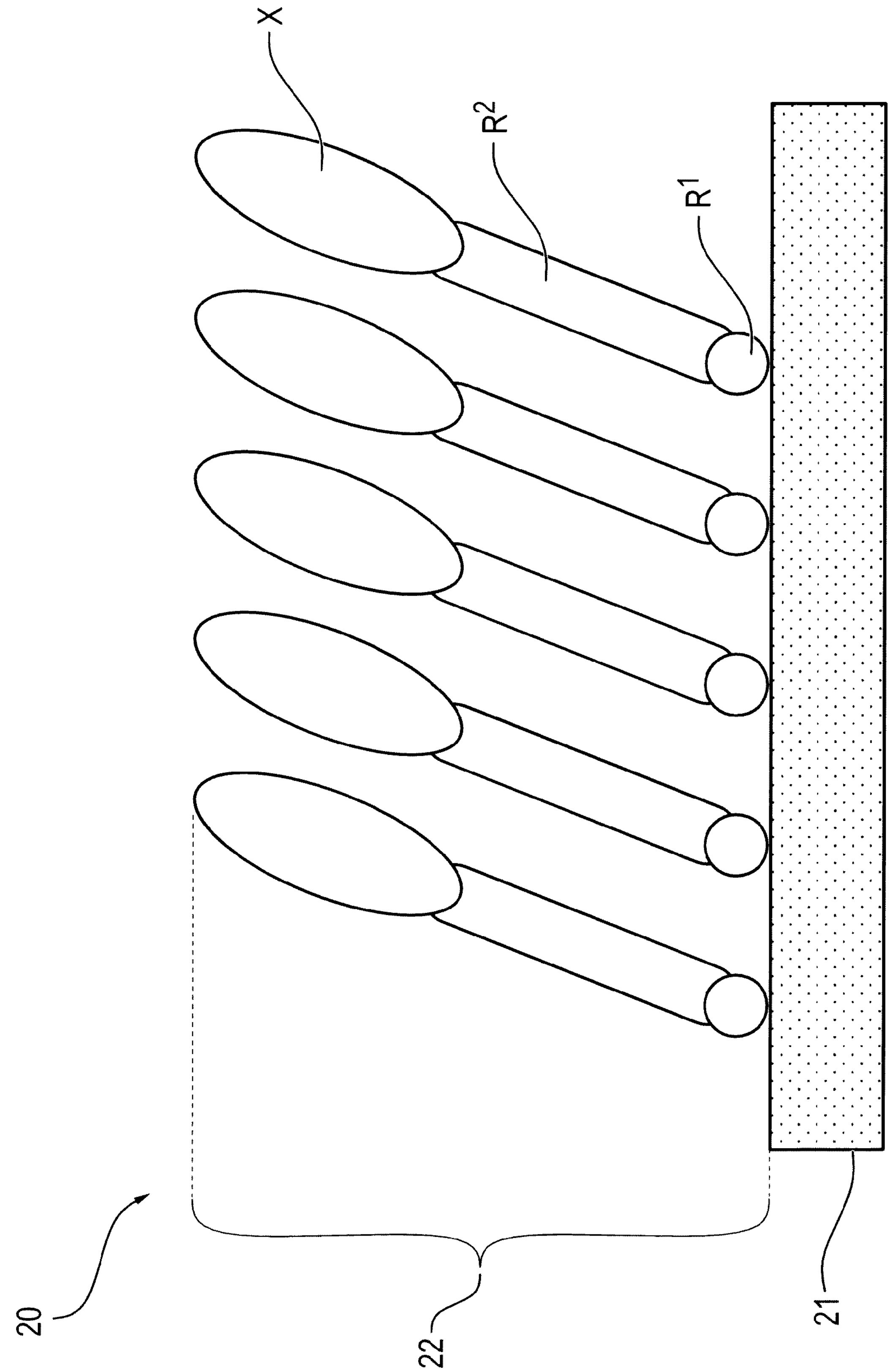


FIG. 2

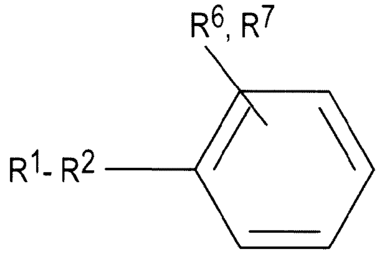

FIG. 3a

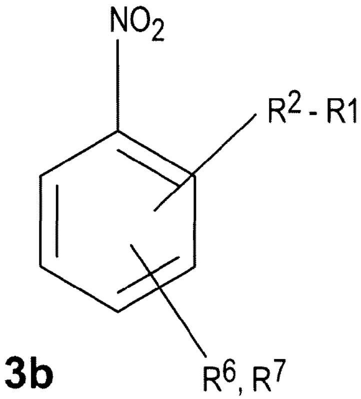

FIG. 3b

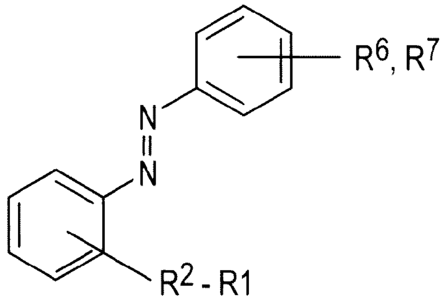

FIG. 3c

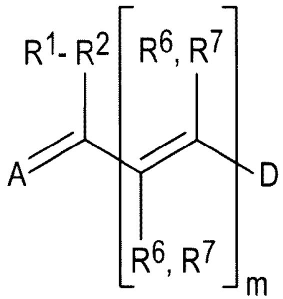

FIG. 3d

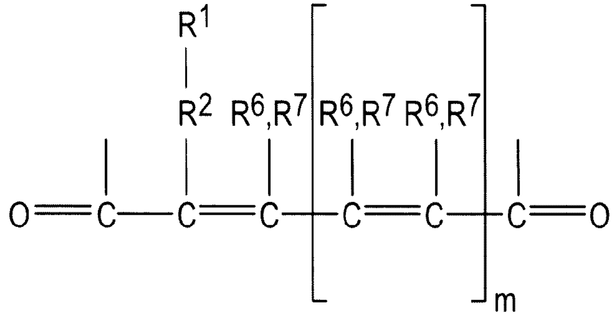

FIG. 3e

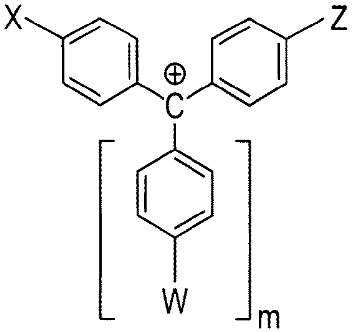

FIG. 3f

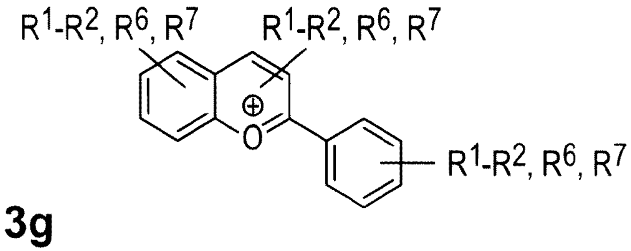

FIG. 3g

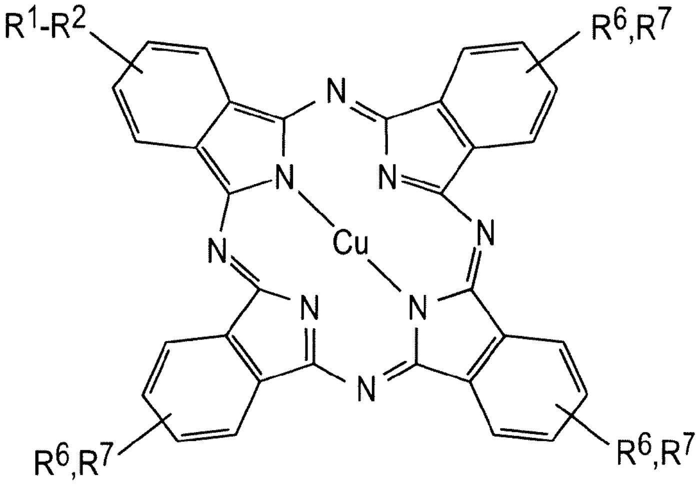

FIG. 3h

FIG. 4

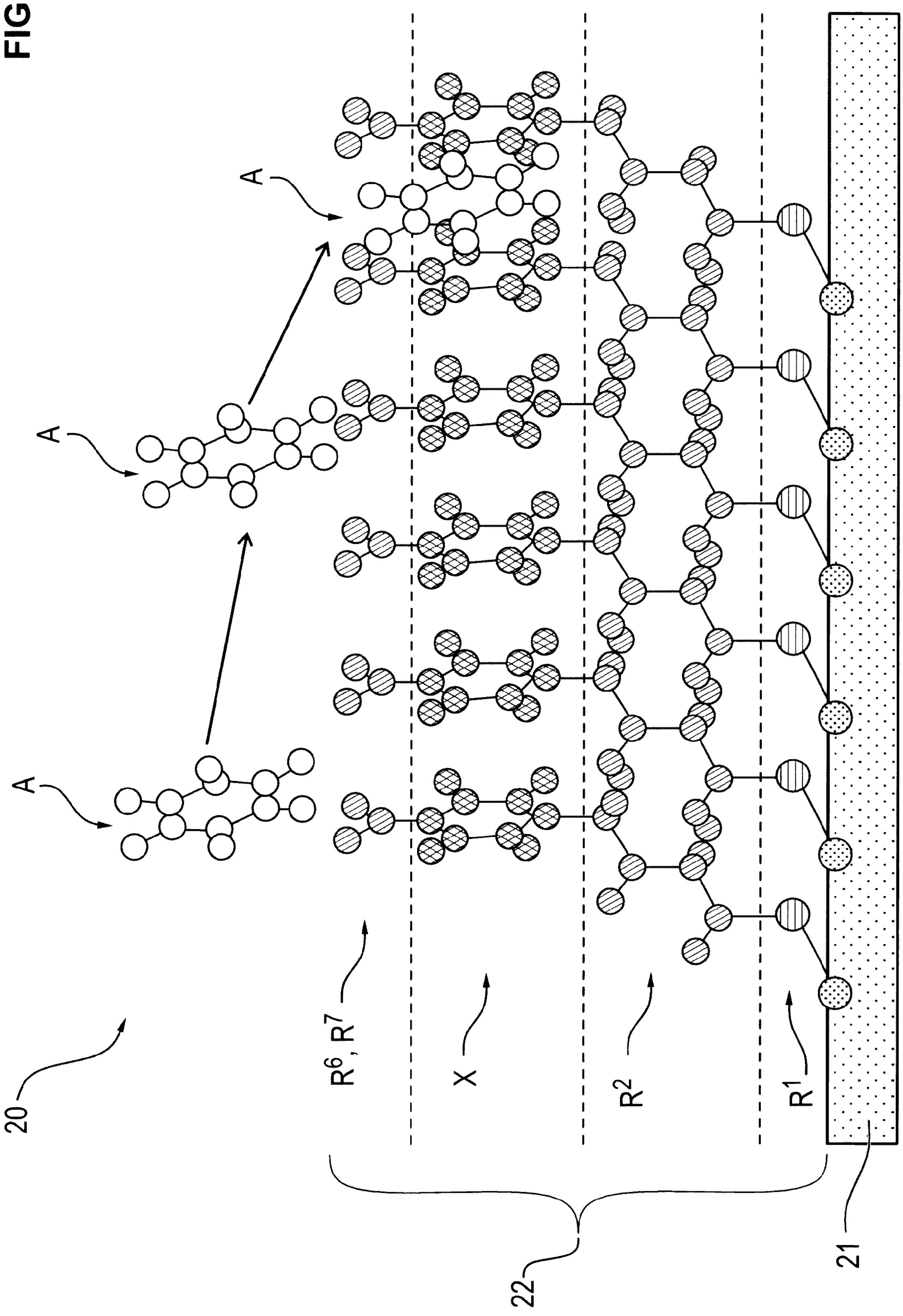

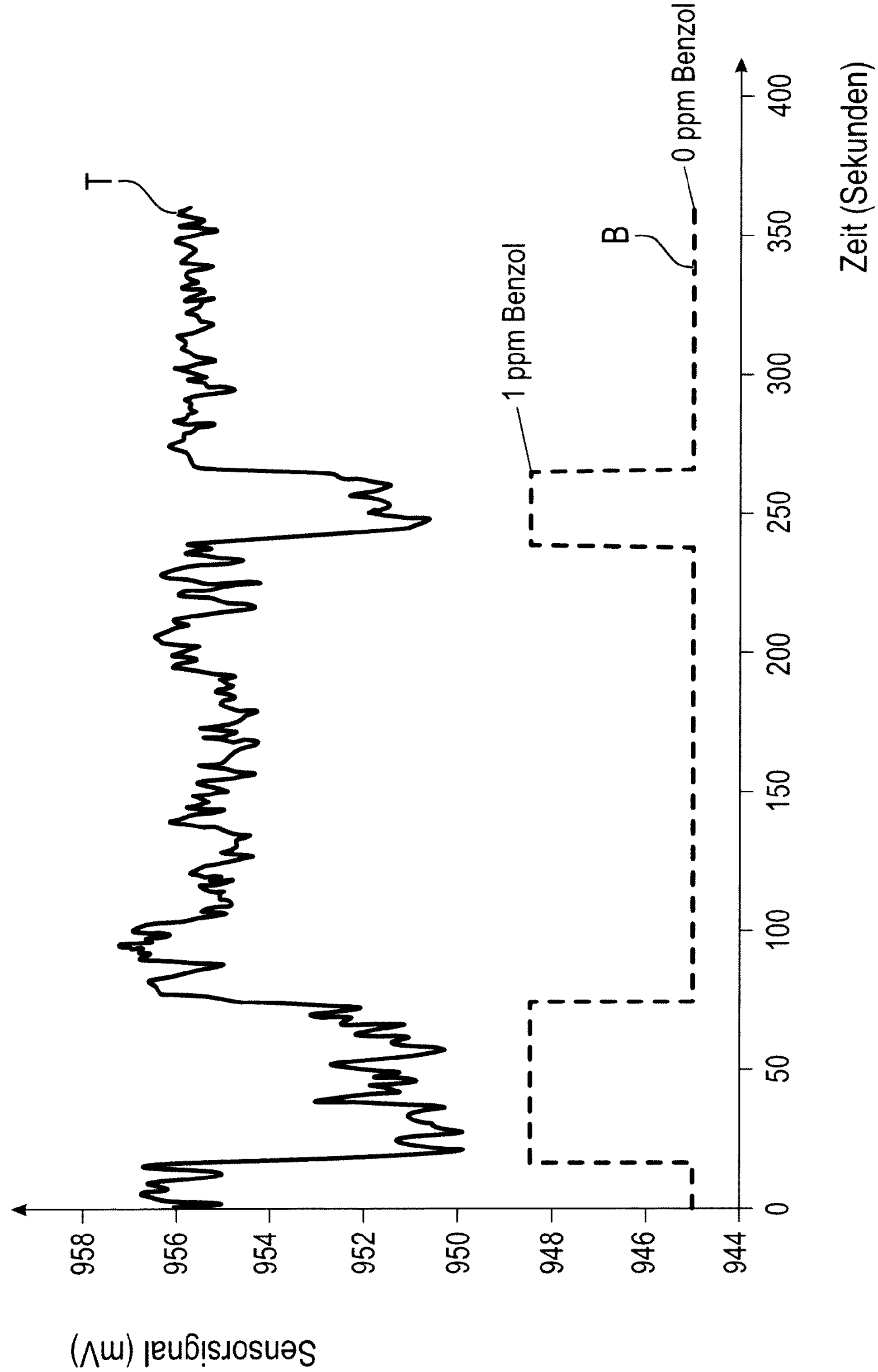
Sensorsignal (mV)
958
956
954
952
950
948
946
944
Zeit (Sekunden)
0
50
100
150
200
250
300
350
400
T
B
1 ppm Benzol
0 ppm Benzol

FIG. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4411741 A
- DE 4333875 A1
- EP 2006668 A1


### In der Beschreibung aufgeführte Nicht-Patentliteratur


- Room temperature benzene gas detection using gated lateral BJT with assembled solvatochromic. **NICHTSDESTOTROTZ.** IMCS 2012 Konferenz